# EUROPÄISCHE PATENTSCHRIFT

(11) **EP 0 804 508 B1**
(45) Veröffentlichungstag und Bekanntmachung des Hinweises auf die Patenterteilung: **09.06.1999**
(21) Anmeldenummer: 96901263.2
(22) Anmeldetag: 12.01.1996
(51) Int. Cl.: C09B 5/62, C07D 221/18

(54) **VERFAHREN ZUR HERSTELLUNG UND REINIGUNG VON PERYLEN-3,4-DICARBONSÄUREIMIDEN**
PROCESS FOR PRODUCING AND PURIFYING PERYLENE-3,4-DICARBOXYLIC ACID IMIDES
PROCEDE DE FABRICATION ET DE PURIFICATION D'IMIDES DE PERYLENE-3,4-ACIDE DICARBOXYLIQUE

(30) Priorität: 20.01.1995 DE 19501737
(43) Veröffentlichungstag der Anmeldung: 05.11.1997
(73) Patentinhaber: BASF AKTIENGESELLSCHAFT, 67056 Ludwigshafen (DE)
(72) Erfinder: BÖHM, Arno, D-68305 Mannheim (DE); HELFER, Willi, D-67159 Friedelsheim (DE)
(86) Internationale Anmeldenummer: EP9600117
(87) Internationale Veröffentlichungsnummer: WO9622331

(56) Entgegenhaltungen:
- EP-A- 0 657 436
- DE-A- 4 236 885
- BULLETIN OF THE CHEMICAL SOCIETY OF JAPAN, Bd. 54, 1981, TOKYO JP, Seiten 1575-1576, XP002003213 Y.NAGAO ET AL: "Synthesis and Reactions of Perylenecarboxylic Acid Derivatives. X." in der Anmeldung erwähnt

## Beschreibung

Die vorliegende Erfindung betrifft ein neues Verfahren zur Herstellung von Perylen-3,4-dicarbonsäureimiden durch Umsetzung einer Perylen-3,4,9,10-tetracarbonsäure oder der entsprechenden Anhydride mit einem primären Amin.

Weiterhin betrifft die Erfindung ein neues Verfahren zur Reinigung von Perylen-3,4-dicarbonsäureimiden, die durch Umsetzung einer Perylen-3,4,9,10-tetracarbonsäure oder der entsprechenden Anhydride mit einem primären Amin erhalten worden sind.

Schließlich betrifft die Erfindung neue Perylen-3,4-dicarbonsäureimide der allgemeinen Formel Ia 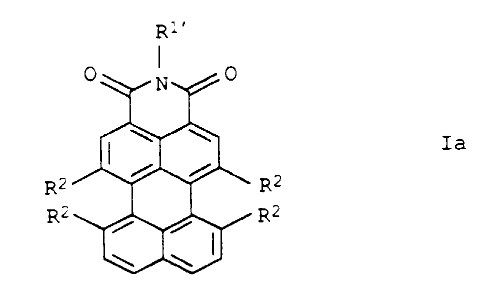 in der die Variablen folgende Bedeutung haben:
- R¹': C₁₄-C₃₀-Alkyl, dessen Kohlenstoffkette durch eine oder mehrere Gruppierungen -O-, -S-, -NR³⁻, -CO- und/oder -SO₂- unterbrochen sein kann und das durch Carboxy, Sulfo, Hydroxy, Cyano, C₁-C₆-Alkoxy oder einen über ein Stickstoffatom gebundenen 5- bis 7-gliedrigen heterocyclischen Rest, der weitere Heteroatome enthalten und aromatisch sein kann, ein- oder mehrfach substituiert sein kann, wobei
R³ Wasserstoff oder C₁-C₆-Alkyl bedeutet; C₅-C₈-Cycloalkyl, dessen Kohlenstoffgerüst durch eine oder mehrere Gruppierungen -O-, -S- und/oder -NR³- unterbrochen sein kann;
Phenyl, das durch C₁-C₄-Alkyl oder Methoxy mindestens in beiden ortho-Positionen, durch C₅-C₁₈-Alkyl, C₂-C₆-Alkoxy, Halogen, Hydroxy, Cyano, Carboxy, -CONHR⁴, -NHCOR⁴ und/oder Aryl- oder Hetarylazo, das jeweils durch C₁-C₁₀-Alkyl, C₁-C₆-Alkoxy, Halogen, Hydroxy, Cyano oder Carboxy substituiert sein kann, ein- oder mehrfach substituiert ist, wobei
R⁴ Wasserstoff; C₁-C₁₈-Alkyl; Aryl oder Hetaryl, das jeweils durch C₁-C₆-Alkyl, C₁-C₆-Alkoxy, Halogen, Hydroxy oder Cyano substituiert sein kann, bedeutet; Naphthyl oder Hetaryl, das jeweils durch die für Phenyl genannten Substituenten substituiert sein kann, wobei die C₁-C₄-Alkyl- und C₁-C₆-Alkoxysubstituenten beliebige Positionen am Ringsystem einnehmen können;
- R²: unabhängig voneinander Wasserstoff; Halogen; C₁-C₁₈-Alkyl; Aryloxy, Arylthio, Hetaryloxy oder Hetarylthio, das jeweils durch C₁-C₁₀-Alkyl, C₁-C₆-Alkoxy, Cyano oder Carboxy substituiert sein kann.

Perylen-3,4-dicarbonsäureimide der Formel 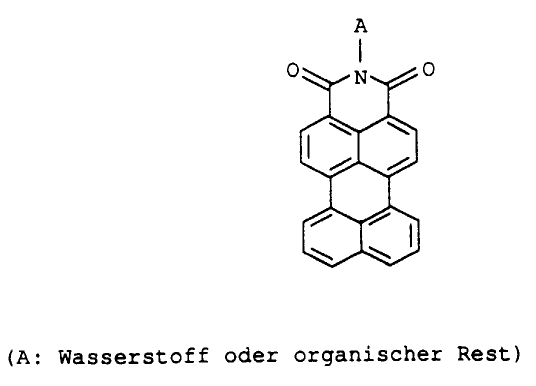 eignen sich bekanntermaßen als Zwischenprodukte für die Herstellung von Pigmentadditiven, Fluoreszenzfarbstoffen und Fluoreszenzpigmenten (DE-A-43 25 247; EP-A-596 292; Chimia 48, 503-505 (1994)).

Neben dem unsubstituierten Perylen-3,4-dicarbonsäureimid (A-H) sind bislang einige wenige N-alkyl- und N-phenyl-substituierte Perylen-3,4-dicarbonsäureimide (A = Methyl, Ethyl, n-Propyl, n-Butyl, Isobutyl, n-Pentyl, n-Hexyl, n-Octyl, n-Dodecyl, Phenyl, 4-Tolyl, 4-Anisyl, 2,5-Di-tert.-butylphenyl) bekannt, die nach aufwendigen, mehrstufigen Verfahren, ausgehend von Perylen-3, 4,9,10-tetracarbonsäuredianhydrid 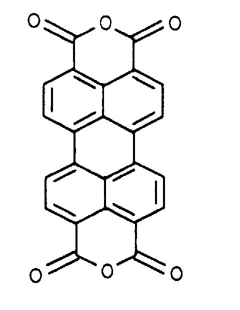 über die entsprechenden Perylen-3,4,9,10-tetracarbonsäureimidanhydride 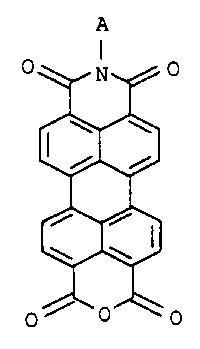 in zumeist wenig befriedigenden Ausbeuten und Reinheiten, die aufwendige Reinigungsmethoden (Extraktion, Säulenchromatographie) erforderlich machen, hergestellt werden.

So werden das unsubstituierte und die N-alkylsubstituierten Perylen-3,4-dicarbonsäureimide durch alkalische Decarboxylierung der Imidanhydride bei Temperaturen ≥ 220°C unter Druck (Reaktionszeiten von 18 h) erhalten (DE-PS 486 491; Bulletin of the Chemical Society of Japan 54, 1575-1576 (1981); EP-A 596 292). Dieses Verfahren ist jedoch nur für gegenüber Basen stabile aliphatische Imide geeignet.

Zur Herstellung der N-phenyl-, -tolyl- und -anisylsubstituierten (sowie auch N-methyl- und -ethylsubstituierten) Perylen-3,4-dicarbonsäureimide wird das zunächst hergestellte unsubstituierte Perylen-3,4-dicarbonsäureimid mit Schwefelsäure sulfoniert, anschließend mit Kalilauge in das sulfonierte Anhydrid überführt, welches dann mit dem entsprechenden primären Amin zu dem sulfonierten N-substituierten Imid umgesetzt wird, welches schließlich mit Schwefelsäure zum gewünschten Perylen-3,4-dicarbonsäureimid desulfoniert wird (Bulletin of the Chemical Society of Japan 52, 1723-1726 (1979), Shikizai Kyokaishi 49, 29-34 (1976) ≙ Chemical Abstracts 85:20928). Dieses Verfahren ist sehr umständlich und kann nur für gegenüber Schwefelsäure stabile Imide eingesetzt werden.

Schließlich wird in Chimia 48, 502-505 (1994) erwähnt, daß N-(2,5-Di-tert.-butylphenyl)perylen-3,4-dicarbonsäureimid durch Kondensation von Perylen-3,4,9,10-tetracarbonsäuredianhydrid mit 2,5-Di-tert.-butylanilin in Gegenwart von Wasser erhalten werden kann. Diese Reaktion liefert das Imid jedoch ebenfalls nur in einer Ausbeute von 50 %, außerdem können auf diese Weise nur mäßig sterisch gehinderte Amine umgesetzt werden.

Nach diesem Verfahren werden in der EP-A-657 436 (Dokument nach Artikel 54(3) EPÜ für die benannten Vertragsstaaten CH, DE, FR, GB, LI) auch N-(4-tert.-Butylphenyl)-, -(1-Hexylheptyl)- und -(1-Octylnonyl)perylen-3,4-dicarbonsäureimid in geringer Ausbeute erhalten. Weiterhin wird hier die Herstellung von N-(1-nonyldecyl)-, -(C₅-C₈-cycloalkyl)- und -cyclododecylsubstituiertem Perylen-3,4-dicarbonsäureimid ausgehend von Perylen-3,4-dicarbonsäureanhydrid sowie von N-(1-hexyl)-, -(1-octyl)-, (7-tridecyl)- und -(1-tetradecyl)-substituiertem Perylen-3,4-dicarbonsäureimid ausgehend vom unsubistituiertem Imid beschrieben.

Der Erfindung lag die Aufgabe zugrunde, ein einfaches und wirtschaftliches Verfahren bereitzustellen, welches die Herstellung beliebiger Perylen-3,4-dicarbonsäureimide in guten Ausbeuten ermöglicht. Eine weitere Aufgabe bestand darin, ein einfaches, kostengünstiges Reinigungsverfahren zu entwickeln, dem die hergestellten Imide, falls erforderlich, zur Erhöhung des Wertgehalts unterzogen werden können.

Demgemäß wurde ein Verfahren zur Herstellung von Perylen-3,4-dicarbonsäureimiden durch Umsetzung einer Perylen-3,4,9,10-tetracarbonsäure oder der entsprechenden Anhydride mit einem primären Amin gefunden, welches dadurch gekennzeichnet ist, daß man die Umsetzung in Gegenwart einer tertiären stickstoffbasischen Verbindung als Lösungsmittel und eines Übergangsmetalls oder Übergangsmetallsalzes als Katalysator vornimmt.

Weiterhin wurde ein Verfahren zur Reinigung von Perylen-3,4-dicarbonsäureimiden, die durch Umsetzung einer Perylen-3,4,9,10tetracarbonsäure oder der entsprechenden Anhydride mit einem primären Amin erhalten worden sind, gefunden, welches dadurch gekennzeichnet ist, daß man die Rohprodukte zunächst in N-Methylpyrrolidon erhitzt und die gebildeten N-Methylpyrrolidon-Addukte dann in Gegenwart eines organischen Verdünnungsmittels mit einer Base behandelt und die danach isolierten Produkte gewünschtenfalls einer zusätzlichen Behandlung mit einer wäßrigen Säure unterzieht.

Außerdem wurde ein Verfahren zur Reindarstellung von Perylen-3,4-dicarbonsäureimiden gefunden, bei dem das Herstellungsverfahren mit diesem Reinigungsverfahren kombiniert wird.

Nicht zuletzt wurden die Perylen-3,4-dicarbonsäureimide der eingangs definierten Formel Ia gefunden.

Bevorzugte Perylen-3,4-dicarbonsäureimide Ia sind den Unteransprüchen zu entnehmen.

Das erfindungsgemäße Verfahren eignet sich zur Herstellung beliebiger, in 9,10-Stellung nicht substituierter Perylen-3,4-dicarbonsäureimide. Beispielsweise können Perylen-3,4-dicarbonsäureimide der allgemeinen Formel I 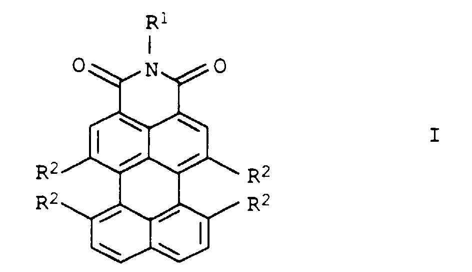 in der die Variablen folgende Bedeutung haben, auf vorteilhafte Weise erhalten werden:
- R¹: Wasserstoff;
C₁-C₃₀-Alkyl, dessen Kohlenstoffkette durch eine oder mehrere Gruppierungen -O-, -S-, -NR³⁻, -CO- und/oder -SO₂- unterbrochen sein kann und das durch Carboxy, Sulfo, Hydroxy, Cyano, C₁-C₆-Alkoxy oder einen über ein Stickstoffatom gebundenen 5- bis 7-gliedrigen heterocyclischen Rest, der weitere Heteroatome enthalten und aromatisch sein kann, ein- oder mehrfach substituiert sein kann, wobei
R³ Wasserstoff oder C₁-C₆-Alkyl bedeutet; C₅-C₈-Cycloalkyl, dessen Kohlenstoffgerüst durch eine oder mehrere Gruppierungen -O-, -S- und/oder -NR³- unterbrochen sein kann;
Aryl oder Hetaryl, das jeweils durch C₁-C₁₈-Alkyl,
C₁-C₆-Alkoxy, Halogen, Hydroxy, Cyano, Carboxy, -CONHR⁴, -NHCOR⁴ oder Aryl- und/oder Hetarylazo, das jeweils durch C₁-C₁₀-Alkyl, C₁-C₆-Alkoxy, Halogen, Hydroxy, Cyano oder Carboxy substituiert sein kann, ein- oder mehrfach substituiert sein kann, wobei
R⁴ Wasserstoff; C₁-C₁₈-Alkyl; Aryl oder Hetaryl, das jeweils durch C₁-C₆-Alkyl, C₁-C₆-Alkoxy, Halogen, Hydroxy oder Cyano substituiert sein kann, bedeutet;
- R²: unabhängig voneinander Wasserstoff; Halogen; C₁-C₁₈-Alkyl; Aryloxy, Arylthio, Hetaryloxy oder Hetarylthio, das jeweils durch C₁-C₁₀-Alkyl, C₁-C₆-Alkoxy, Cyano oder Carboxy substituiert sein kann.

Besondere Bedeutung hat das erfindungsgemäße Verfahren für die Herstellung der neuen, eingangs definierten und der den Unteransprüchen zu entnehmenden, bevorzugten Perylen-3,4-dicarbonsäureimide Ia.

So können Amine mit höherer Alkylkettenlänge (in der Regel > C₁₂), die in den bei den bekannten Verfahren eingesetzten wäßrigen Reaktionsmedien zur Unlöslichkeit der Imide führen würden, Amine mit modifizierten und/oder substituierten Alkylketten sowie auch aromatischen Resten, die unter den bisher verwendeten Reaktionsbedingungen (stark basisch bzw. sauer) nicht stabil gewesen wären, und vor allem auch sterisch gehinderte aromatische Amine, insbesondere o,o'-disubstituierte Aniline, die nach den bekannten Verfahren ebenfalls nicht eingesetzt werden können, problemlos mit den entsprechenden Perylen-3,4,9,10-tetracarbonsäuren bzw. deren Anhydriden, insbesondere Dianhydriden, welche die Substituenten R² tragen, umgesetzt werden.

Alle in den Formeln I und Ia auftretenden Alkylgruppen können sowohl geradkettig als auch verzweigt sein. Aromatische Reste, die substituiert sind, können im allgemeinen bis zu 3, bevorzugt 1 oder 2 der genannten Substituenten aufweisen.

Als Beispiele für geeignete Reste R¹ sowie R² (bzw. für deren Substituenten) seien im einzelnen genannt:

Methyl, Ethyl, Propyl, Isopropyl, Butyl, Isobutyl, sec-Butyl, tert-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen - vgl, dazu Ullmanns Encyklopädie der technischen Chemie, 4. Auflage, Band 7, Seiten 215 bis 217, sowie Band 11, Seiten 435 und 436);

2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3, 6, 9-Trioxadodecyl, 3,6,9,12-Tetraoxatridecyl und 3,6,9,12-Tetraoxatetradecyl;

2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 4,7-Dithiaoctyl, 4,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;

2-Monomethyl- und 2-Monoethylaminoethyl, 2-Dimethylaminoethyl, 2- und 3-Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;

Propan-2-on-1-yl, Butan-3-on-1-yl, Butan-3-on-2-yl und 2-Ethylpentan-3-on-1-yl;

2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl;

Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;

Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;

2-Hydroxyethyl, 2-Hydroxypropyl, 1-Hydroxyprop-2-yl, 2- und 4-Hydroxybutyl, 1-Hydroxybut-2-yl und 8-Hydroxy-4-oxaoctyl, 2-Cyanoethyl, 3-Cyanopropyl, 2-Methyl-3-ethyl-3-cyanopropyl, 7-Cyano-7-ethylheptyl und 4-Methyl-7-methyl-7-cyanoheptyl;

Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;

Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;

Formylamino, Acetylamino, Propionylamino und Benzoylamino; Chlor, Brom und Iod;

Phenylazo, 2-Naphthylazo, 2-Pyridylazo und 2-Pyrimidylazo;

Cyclopentyl, Cyclohexyl, Cycloheptyl, Cyclooctyl, 2-Dioxanyl, 4-Morpholinyl, 2- und 3-Tetrahydrofuryl, 1-, 2- und 3-Pyrrolidinyl und 1-, 2-, 3- und 4-Piperidyl;

Phenyl, 2-Naphthyl, 2- und 3-Pyrryl, 2-, 3- und 4-Pyridyl, 2-, 4-und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5-Isochinolyl;

2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3- und 4-Butylphenyl, 2,4-, 3,5- und 2,6-Dibutylphenyl, 2,4,6-Tributylphenyl, 2-, 3- und 4-Isobutylphenyl, 2,4-, 3,5- und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec-butylphenyl; 2-, 3-und 4-Methoxyphenyl, 2,4-, 3,5- und 2,6-Dimethoxyphenyl, 2,4,6-Trimethoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3- und 4-Isopropoxyphenyl, 2,4- und 2,6-Diisopropoxyphenyl und 2-, 3- und 4-Butoxyphenyl; 2-, 3- und 4-Chlorphenyl und 2,4-, 3,5- und 2,6-Dichlorphenyl; 2-, 3- und 4-Hydroxyphenyl und 2,4-, 3,5- und 2,6-Dihydroxyphenyl; 2-, 3- und 4-Cyanophenyl; 3- und 4-Carboxyphenyl; 3- und 4-Carboxyamidophenyl, 3- und 4-N-Methylcarboxamidophenyl und 3- und 4-N-Ethylcarboxamidophenyl; 3- und 4-Acetylaminophenyl, 3- und 4-Propionylaminophenyl und 3- und 4-Butyrylaminophenyl; 3- und 4-N-Phenylaminophenyl, 3- und 4-N-(o-Tolyl)aminophenyl, 3- und 4-N-(m-Tolyl)aminophenyl und 3- und 4-(p-Tolyl)aminophenyl; 3- und 4-(2-Pyridyl)aminophenyl, 3- und 4-(3-Pyridyl)aminophenyl, 3- und 4-(4-Pyridyl)aminophenyl, 3-und 4-(2-Pyrimidyl)aminophenyl und 4-(4-Pyrimidyl)aminophenyl;

4-Phenylazophenyl, 4-(l-Naphthylazo)phenyl, 4-(2-Naphthylazo)phenyl, 4-(4-Naphthylazo)phenyl, 4-(2-Pyridylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;

Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.

Beim erfindungsgemäßen Verfahren zur Herstellung von Perylen-3,4-dicarbonsäureimiden werden als Edukte die entsprechend substituierten Perylen-3,4,9,10-tetracarbonsäuren bzw. deren Anhydride, insbesondere die Dianhydride, eingesetzt, die ihrerseits durch Halogenierung und gewünschtenfalls anschließenden Austausch der Halogenatome durch Aryloxy-, Arylthio-, Hetaryloxy-, Hetarylthio- oder Alkylreste erhalten werden können.

Die besonders interessanten, 1,7-disubstituierten Perylen-3,4,9,10-tetracarbonsäuren bzw. Anhydride sind dabei, wie in den älteren deutschen Patentanmeldungen 195 47 209.8 und 195 47 210.1 beschrieben, nach einem mehrstufigen Verfahren ausgehend von durch selektive Bromierung von Perylen-3,4,9,10-tetracarbonsäure bzw. -dianhydrid in 100 gew.-%iger Schwefelsäure bei 80 bis 90°C hergestellter 1,7-Dibromperylen-3,4,9,10-tetracarbonsäure bzw. deren Dianhydrid erhältlich. Diese werden in Gegenwart eines polaren aprotischen Lösungsmittels wie N-Methylpyrrolidon und gegebenenfalls eines Imidierungskatalysators, z.B. einer organischen oder anorganischen Säure oder eines Übergangsmetallsalzes, mit einem primären Amin zum entsprechenden 1,7-Dibromperylen-3,4,9,10-tetracarbonsäurediimid umgesetzt, welches anschließend entweder in Gegenwart eines inerten aprotischen Lösungsmittels wie N-Methylpyrrolidon und einer nicht oder nur schwach nucleophilen Base, z.B. Natrium- oder Kaliumcarbonat, mit einem aromatischen Alkohol oder Thioalkohol oder aber in Gegenwart eines aprotischen Lösungsmittels wie Tetrahydrofuran, eines Palladiumkomplexes als Katalysator sowie eines Kupfersalzes als Cokatalysator und einer Base, z.B. Piperidin, mit einem 1-Alkin umgesetzt wird. Im letztgenannten Fall werden 1,7-disubstituierte Perylen-3,4,9,10-tetracarbonsäurediimide erhalten, die im Substituenten R² ungesättigte Bindungen enthalten, die durch Nachrühren in einer Wasserstoffatmosphäre oder durch katalytische Reduktion mit Wasserstoff reduziert werden können. In einem letzten Reaktionsschritt wird das entweder 1,7-diaroxy-, -diarylthio- oder -dialkylsubstituierte Perylen-3,4,9,10-tetracarbonsäurediimid dann in Gegenwart eines polaren protischen Lösungsmittels wie Isopropanol und einer Base, z.B. Natrium- oder Kaliumhydroxid, zu der 1,7-disubstituierten Perylen-3,4,9,10-tetracarbonsäure bzw. deren Dianhydrid verseift.

Beim erfindungsgemäßen Herstellungsverfahren werden die Perylen-3,4,9,10-tetracarbonsäuren bzw. deren Anhydride, insbesondere die Dianhydride, mit den gewünschten primären Aminen (vor allem R¹-NH₂) in Gegenwart einer tertiären stickstoffbasischen Verbindung als Lösungsmittel und eines Übergangsmetalls oder Übergangsmetallsalzes als Katalysator umgesetzt.

Hierbei laufen sowohl eine einseitige Kondensations- (Imidierungs-)reaktion als auch eine einseitige Decarboxylierungsreaktion ab.

Als Lösungsmittel eignen sich insbesondere solche tertiären Stickstoffbasen, deren Schmelzpunkt unterhalb Raumtemperatur liegt, da auf diese Weise die Aufarbeitung des Reaktionsgemisches sowie die Rückgewinnung des Lösungsmittels erleichtert wird.

Beispiele für geeignete Basen sind cyclische Imide wie N-Methylpyrrolidon, tertiäre aliphatische Amine NR³, deren Alkylreste R 4 bis 8 C-Atome aufweisen, wie Trihexylamin, und vor allem aromatische Heterocyclen wie Chinaldin, Isochinolin und insbesondere Chinolin.

Die Menge an Lösungsmittel ist an sich nicht kritisch, üblicherweise kommen 2 bis 20 kg, vorzugsweise 6 bis 12 kg, Lösungsmittel je kg Perylen-3,4,9,10-tetracarbonsäuredianhydrid zum Einsatz.

Als Katalysatoren eignen sich insbesondere die Übergangsmetalle Eisen und vor allem Zink und Kupfer sowie besonders auch deren anorganische und organische Salze, die vorzugsweise in wasserfreier Form eingesetzt werden.

Beispiele für bevorzugte Salze sind Kupfer(I)oxid, Kupfer(II)oxid, Kupfer(I)chlorid, Kupfer(II)acetat, Zinkacetat und Zinkpropionat.

Selbstverständlich kann man auch Mischungen der genannten Katalysatoren verwenden.

In der Regel kommen 5 bis 80 Gew.-% Katalysator, bezogen auf das Perylen-3,4,9,10-tetracarbonsäuredianhydrid, zum Einsatz. Bevorzugte Mengen betragen bei den Kupferverbindungen 10 bis 25 Gew.-% und bei den Zinksalzen 40 bis 60 Gew.-%, ebenfalls bezogen auf das Anhydrid.

Als primäre Amine können bei dem erfindungsgemäßen Herstellungsverfahren alle bei der Reaktionstemperatur stabilen primären Amine, vorzugsweise solche, deren Siedepunkt beim Reaktionsdruck oberhalb der Reaktionstemperatur liegt, eingesetzt werden.

Die Reaktionstemperatur beträgt im allgemeinen 120 bis 250°C, insbesondere 170 bis 235°C. Es empfiehlt sich, unter Verwendung einer Schutzgasatmosphäre (z.B. Stickstoff) zu arbeiten.

Man kann das erfindungsgemäße Herstellungsverfahren bei Normaldruck oder bei einem Überdruck von üblicherweise bis zu 10 bar durchführen. Die Arbeitsweise unter Druck ist vor allem beim Einsatz flüchtiger Amine (d.h., mit einem Siedepunkt ≤ etwa 180°C) zweckmäßig.

In der Regel liegt das Molverhältnis der Ausgangsverbindungen Amin und Anhydrid bei 0,8:1 bis 6:1. Bei druckloser Fahrweise (d.h. Normaldruck) beträgt es vorzugsweise 0,8:1 bis 1,2:1, bei Umsetzung unter erhöhtem Druck insbesondere 2:1 bis 4:1.

Üblicherweise ist die erfindungsgemäße Umsetzung in 2 bis 30 h, vor allem in 3 bis 12 h, beendet.

Bei druckloser Arbeitsweise geht man verfahrenstechnisch zweckmäßigerweise wie folgt vor:

Man legt Perylen-3,4,9,10-tetracarbonsäuredianhydrid und Katalysator in einem Teil der Lösungsmittelmenge (z.B. etwa der Hälfte) vor, spült die Apparatur mit Stickstoff (etwa 15 min), erhitzt das Gemisch unter Rühren auf die Reaktionstemperatur und tropft das primäre Amin, gelöst in der restlichen Lösungsmittelmenge, in etwa 2 bis 6 h zu. Nach einer Nachrührzeit von üblicherweise etwa 0,5 bis 8 h bei der Reaktionstemperatur kühlt man auf 120 bis 140°C ab und filtriert bei dieser Temperatur noch nicht umgesetztes Anhydrid sowie den größten Teil des Katalysators ab.

Die weitere Aufarbeitung des auf Raumtemperatur abgekühlten Filtrats auf die (Roh-)Produkte (Perylen-3,4-dicarbonsäureimid, verunreinigt durch wechselnde Mengen Diimid) kann man in üblicher Weise vornehmen, indem man, gegebenenfalls nach Zugabe von primären Alkoholen wie Methanol zur Vervollständigung der Ausfällung, die ausgefallenen Produkte abfiltriert, wäscht und trocknet.

Werden Perylen-3,4-dicarbonsäureimide hergestellt, die bei Temperaturen ≤ etwa 140°C nicht mehr im Lösungsmittel löslich sind (z.B. N-(4-Phenylazophenyl)perylen-3,4-dicarbonsäureimid), so filtriert man diese Imide zweckmäßigerweise bei dieser Temperatur ab, wäscht den Filterkuchen vorzugsweise mit heißem (ebenfalls etwa 130°C) Lösungsmittel und Methanol und kocht ihn etwa 0,5 bis 1 h zur vollständigen Entfernung des Katalysators in verdünnter anorganischer Säure (z.B. 10 bis 15 gew.-%iger Salzsäure). Anschließend kann man die Imide wie üblich durch Filtration der abgekühlten Mischung, Waschen mit Wasser bis zur Neutralität und Salzfreiheit des ablaufenden Waschwassers und Trocknen isolieren.

In der Regel haben die so behandelten Produkte bereits einen so hohen Wertgehalt (> 95 %), daß eine weitere Reinigung nicht erforderlich ist.

Bei Reaktionsführung unter Druck geht man verfahrenstechnisch zweckmäßigerweise so vor, daß man Perylen-3,4,9,10-tetracarbonsäuredianhydrid, Katalysator und primäres Amin in der gesamten Lösungsmittelmenge vorlegt, die Druckapparatur mit Stickstoff spült (etwa 15 min) und dann verschließt, einen Stickstoffdruck von in der Regel 1 bis 2 bar einstellt und anschließend das Gemisch unter Rühren auf die Reaktionstemperatur erhitzt und etwa 6 bis 8 h bei dieser Temperatur hält. Nach Abkühlen auf üblicherweise 120 bis 140°C und Entspannen kann man das Reaktionsgemisch wie oben beschrieben aufarbeiten.

Genügen die beim erfindungsgemäßen Herstellungsverfahren erhaltenen (Roh-)Produkte nicht den gewünschten Reinheitsanforderungen (in der Regel werden Produkte mit Wertgehalten ≥ 80 % erhalten), so können sie noch dem erfindungsgemäßen Reinigungsverfahren unterzogen werden.

Beim erfindungsgemäßen Reinigungsverfahren werden die Perylen-3,4-dicarbonsäureimid-Rohprodukte zunächst durch Erhitzen in N-Methylpyrrolidon (kurz: NMP) in NMP-Addukte überführt. Erfolgte die Herstellung des Imids bereits in NMP als Lösungsmittel, kann dieser Schritt selbstverständlich entfallen.

Die NMP-Addukte werden anschließend einer alkalischen Reinigungsbehandlung unterzogen. Gewünschtenfalls kann noch eine saure Nachbehandlung angeschlossen werden.

Beim ersten Schritt des erfindungsgemäßen Reinigungsverfahrens, der Bildung der NMP-Addukte, geht man üblicherweise so vor, daß man das getrocknete Rohprodukt in der etwa 3- bis 10fachen, vorzugsweise 5,5- bis 6,5fachen, Gewichtsmenge NMP unter Rühren auf etwa 140 bis 200°C, bevorzugt 160 bis 180°C, besonders bevorzugt 165 bis 170°C, erhitzt und in der Regel 10 bis 60 min, insbesondere 15 bis 30 min, bei dieser Temperatur hält. Vorteilhaft führt man diese Behandlung unter Schutzgas (z.B. Stickstoff) durch.

Zweckmäßigerweise kühlt man das Gemisch dann, bevorzugt unter langsamem Rühren, auf zunächst etwa 50 bis 55°C und dann, ohne Rühren, auf Raumtemperatur ab.

Das NMP-Addukt kann dann in üblicher Weise isoliert werden, indem man es abfiltriert, wäscht (vorzugsweise zunächst mit einer Mischung aus NMP und einem wasserlöslichen Alkohol wie Ethanol, dann mit einer verdünnten Salzsäure und abschließend mit Wasser) und gewünschtenfalls trocknet.

Der zweite Schritt des erfindungsgemäßen Reinigungsverfahrens, die alkalische Behandlung des NMP-Addukts, wird vorteilhaft in Gegenwart eines organischen Reaktionsmediums durchgeführt.

Als organische Verdünnungsmittel eignen sich neben inerten aromatischen Lösungsmitteln wie Toluol und Xylol vorzugsweise Alkohole, die ein- oder mehrwertig sein können, z.B. aromatische Alkohole wie Phenol und Benzylalkohol und aliphatische Alkohole, sowohl Glykole und Glykolether, wie Ethylenglykol, Propylenglykol und Butylglykol (Ethylenglykolmonobutylether) als auch insbesondere C₂-C₆-Alkohole wie Ethanol, Propanol, Butanol, Pentanol und Hexanol, die auch verzweigt sein können, wie bevorzugt Isopropanol.

In der Regel werden 40 bis 200 kg, insbesondere 80 bis 120 kg, Verdünnungsmittel je kg NMP-Addukt eingesetzt.

Geeignete Basen sind neben sterisch gehinderten Stickstoffbasen, wie Diazabicycloundecen und Diazabicyclo[2.2.2]octan, vor allem Alkalimetallhydroxide, wie Natriumhydroxid und insbesondere Kaliumhydroxid, und Alkalimetallsalze sekundärer und tertiärer aliphatischer (bevorzugt C₃-C₆-)Alkohole, wie Natrium-tert.-butylat und insbesondere Kalium-tert.-butylat.

Die Alkalimetallhydroxide werden zweckmäßigerweise zusammen mit Wasser (z.B. 40 bis 60 Gew.-%, bezogen auf das Alkalimetallhydroxid) eingesetzt.

Üblicherweise kommen je kg NMP-Addukt 0,5 bis 15 kg Base zum Einsatz, vorzugsweise 5 bis 7 kg, insbesondere etwa 6 kg, trockenes Alkalimetallhydroxid bzw. 0,5 bis 1,5 kg, insbesondere 0,7 bis 1 kg, Alkoholat oder Stickstoffbase.

Es empfiehlt sich, die alkalische Behandlung in der Wärme vorzunehmen. Im allgemeinen beträgt die Behandlungstemperatur 50 bis 150°C, bevorzugt 60 bis 120°C.

Verfahrenstechnisch geht man zweckmäßigerweise so vor, daß man das Verdünnungsmittel vorlegt, das NMP-Addukt und die Base zugibt, dann das Gemisch unter Rühren auf die Behandlungstemperatur erhitzt und etwa 1 bis 24 h, insbesondere 1 bis 10 h, bei dieser Temperatur hält.

Die Isolierung des gereinigten Produkts kann man wie üblich vornehmen, indem man den Niederschlag nach Abkühlung auf etwa 25 bis 30°C und gegebenenfalls nach Vervollständigung der Fällung durch Zugabe von Methanol abfiltriert, wäscht (bevorzugt mit dem für die Reinigung ausgewählten Verdünnungsmittel, Methanol und Wasser) und trocknet.

Gewünschtenfalls kann man noch eine zusätzliche Säurebehandlung anschließen, bei der man den nicht getrockneten Filterkuchen in einer verdünnten anorganischen Säure, z.B. 4 bis 6 gew.-%iger Salzsäure, (etwa 4 bis 6 kg Säure je kg Filterkuchen) aufrührt.

Anschließend kann das gereinigte Imid wie üblich durch Filtration, Waschen mit Wasser (bis zur Neutralität des ablaufenden Waschwassers) und Trocknen isoliert werden.

Die zusätzliche Säurebehandlung empfiehlt sich insbesondere bei Perylen-3,4-dicarbonsäureimiden der Formel I, in der R¹ Phenyl oder durch C₁-C₄-Alkyl substituiertes Phenyl und R² Wasserstoff bedeutet.

Beim erfindungsgemäßen Verfahren zur Reindarstellung von Perylen-3,4-dicarbonsäureimiden werden das erfindungsgemäße Herstellungsverfahren und das erfindungsgemäße Reinigungsverfahren in vorteilhafter Weise miteinander kombiniert. Hierbei erhält man beliebige Perylen-3,4-dicarbonsäureimide auf verfahrenstechnisch einfache wirtschaftliche Weise in hervorragenden Reinheiten (im allgemeinen > 98 %) und guten Ausbeuten (im allgemeinen 60 bis 90 %).

Die erfindungsgemäßen neuen Perylen-3,4-dicarbonsäureimide Ia (wie auch die erfindungsgemäß hergestellten Imide I) eignen sich vorteilhaft sowohl als Pigmentadditivvorstufen als auch als Pigmente oder Fluoreszenzfarbstoffe. Sie können insbesondere zur Einfärbung von hochmolekularen organischen Materialien oder auch organisch/anorganischen Compositen verwendet werden.

Als Pigmente sind insbesondere die Perylen-3,4-dicarbonsäureimide Ia geeignet, bei denen R¹' Phenyl oder monocyclisches Hetaryl (besonders Pyridyl oder Pyrimidyl), das jeweils durch C₁-C₄-Alkyl und/oder Cyano ein- bis dreifach oder durch Phenylazo oder Naphthylazo einfach substituiert ist, und R² Wasserstoff bedeutet.

Als Fluoreszenzfarbstoffe eignen sich vor allem die Perylen-3,4-dicarbonsäureimide Ia, bei denen R¹' Phenyl oder monocyclisches Hetaryl (besonders Pyridyl oder Pyrimidyl), das jeweils durch Hydroxy, Carboxy, -CONHR⁴ und/oder -NHCOR⁴ (R⁴: C₁-C₄-Alkyl oder Phenyl, das durch C₁-C₄-Alkyl oder C₁-C₄-Alkoxy substituiert sein kann) ein- bis dreifach substituiert ist, oder C₅-C₈-Cycloalkyl und R² Wasserstoff oder Phenoxy, das durch C₁-C₄-Alkyl ein- bis dreifach substituiert sein kann, bedeutet.

### Beispiele

### A) Herstellung von Perylen-3,4-dicarbonsäureimiden der Formel Ib

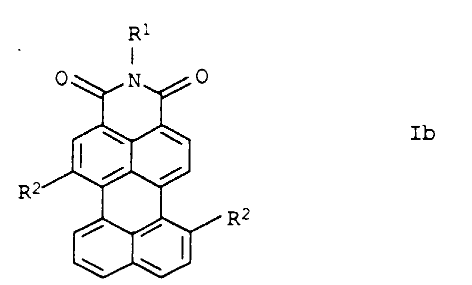

aus den entsprechenden Perylen-3,4,9,10-tetracarbonsäuredianhydriden der Formel II

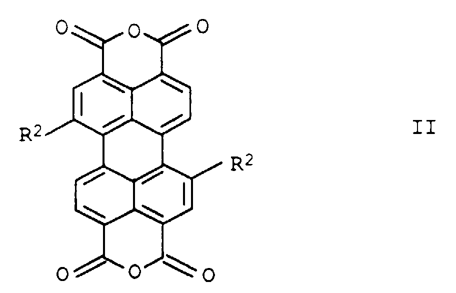

Die als Ausgangsprodukt für die 1,7-disubstituierten Perylen-3,4-dicarbonsäureimide (Beispielen 25 bis 27) eingesetzten 1,7-disubstituierten Perylen-3,4,9,10-tetracarbonsäuredianhydride II wurden wie folgt hergestellt.

### a) Herstellung von 1,7-Dibromperylen-3,4,9,10-tetracarbonsäuredianhydrid (IIa)

### Beispiel 1

Eine Mischung von 292,5 g (0,75 mol) Perylen-3,4,9,10-tetracarbonsäuredianhydrid (Wertgehalt > 98 %) und 4420 g 100 gew.-%iger Schwefelsäure wurde nach 12stündigem Rühren und anschließender Zugabe von 7 g Iod auf 85°C erhitzt. Dann wurden 262,5 g (1,64 mol) Brom in 8 h zugetropft.

Nach Abkühlen auf Raumtemperatur und Verdrängen des überschüssigen Broms durch Stickstoff wurde die Schwefelsäurekonzentration des Reaktionsgemisches durch portionsweise Zugabe von insgesamt 670 g Wasser in 1 h auf 86 Gew.-% erniedrigt. Nach dem Abkühlen des sich dabei auf 85°C erhitzenden Reaktionsgemisches auf Raumtemperatur wurde das ausgefallene Produkt über eine G4-Glasfritte abfiltriert, mit 3 kg 86 gew.-%iger Schwefelsäure gewaschen, dann in 5 1 Wasser aufgerührt, erneut abfiltriert, neutral gewaschen und bei 120°C im Vakuum getrocknet.

Es wurden 370 g IIa in Form eines leuchtendroten, feinkristallinen Pulvers mit einem Schmelzpunkt > 360°C und einem Wertgehalt von >98 % erhalten, was einer Ausbeute von 90 % entspricht.

### b) Herstellung von N,N'-Dicyclohexyl-1,7-dibromperylen-3,4,9,10-tetracarbonsäurediimid (III)

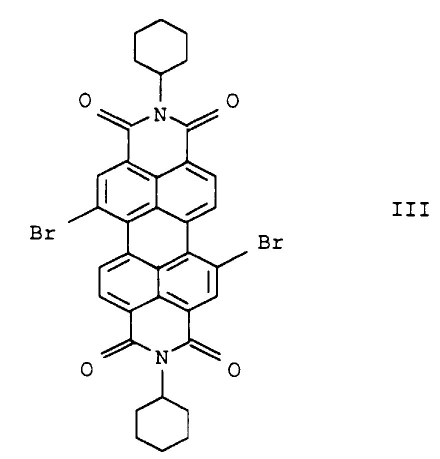

### Beispiel 2

Zu einer Mischung von 69,9 g (127 mmol) 1,7-Dibromperylen-3,4,9,10-tetracarbonsäuredianhydrid (IIa) (Beispiel 1) in 900 ml N-Methyl-2-pyrrolidon wurden unter Rühren zunächst 42,8 g Eisessig und dann portionsweise insgesamt 381 mmol Cyclohexylamin zugegeben. Anschließend wurde das Reaktionsgemisch unter Stickstoff auf 85°C erhitzt und 6 h bei dieser Temperatur gerührt.

Nach dem Abkühlen auf Raumtemperatur wurde das ausgefallene Reaktionsprodukt abfiltriert, mit insgesamt 2 1 Methanol gewaschen und bei 100°C im Vakuum getrocknet.

Es wurden 75,1 g III als hellrotes, mikrokristallines Pulver mit einem Schmelzpunkt >360°C und einem Wertgehalt von 97 % erhalten, was einer Ausbeute von 83% entspricht.

Analytische Daten:
Elementaranalyse (Gew.-% ber./gef.):
C: 60,7/60,3; H: 4,0/4,2; N: 3,9/3,8;O: 9,0/9,3;
Br: 22,4/22,0;
IR (KBr): ν = 1698 (s, C=O), 1655 (s, C=O) cm⁻¹;
UV/VIS (CHCl₃): λₘₐₓ (ε) = 491 (33411), 526 (50033) nm.

### c) Herstellung von 1,7-diaroxysubstituierten N,N'-Dicyclohe-xylperylen-3,4,9,10-tetracarbonsäurediimiden IV

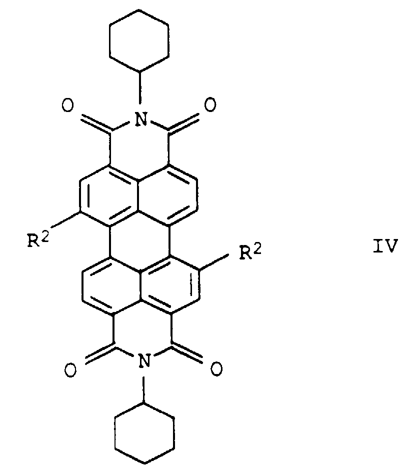

### Beispiele 3 und 4

14,25 g (20 mmol) N,N'-Dicyclohexyl-1,7-dibromperylen-3,4,9,10-tetracarbonsäurediimid (III) aus Beispiel 2 wurden unter Rühren in 450 ml N-Methylpyrrolidon eingetragen, nacheinander mit 6,4 g (46 mmol) wasserfreiem Kaliumcarbonat und a g (40 mmol) des Hydroxyaromaten R²⁻H versetzt und unter Stickstoff 1,5 h auf 120°C erhitzt.

Nach dem Abkühlen auf Raumtemperatur wurde die Reaktionsmischung unter Rühren in 1,5 1 einer 6 gew.-%igen Salzsäure eingetragen. Das ausgefallene Reaktionsprodukt wurde abfiltriert, mit Wasser neutral gewaschen und bei 100°C im Vakuum getrocknet.

Weitere Einzelheiten zu diesen Versuchen sowie deren Ergebnisse sind in Tabelle 1 zusammengestellt.

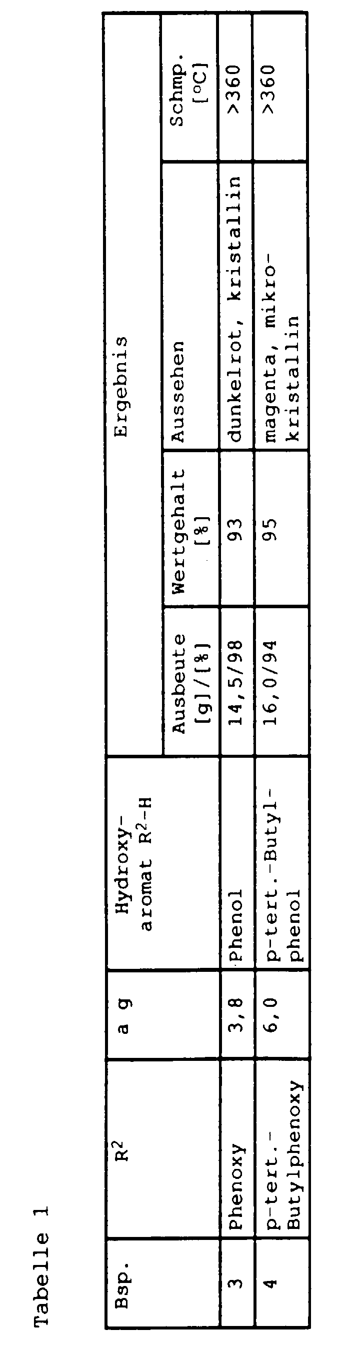

Analytische Daten zu Beispiel 3:
Elementaranalyse (Gew.-% ber./gef.):
C: 78,0/77,5; H: 5,2/5,3; N: 3,8/3,7; O: 13,0/13,4; Masse (FD) : m/z = 738 (M⁺, 100 %);
IR (KBr): ν = 1695 (s, C=O), 1654 (s, C=O) cm⁻¹;
UV/VIS (CHCl₃): λₘₐₓ (ε) = 401 (7455), 513 (37102), 549 (55004) nm.

Analytische Daten zu Beispiel 4:
Elementaranalyse (Gew.-% ber./gef.):
C: 79,0/78,8; H: 6,4/6,4; N: 3,3/3,2; O: 11,3/11,4; Masse (FD): m/z = 850 (M⁺, 100 %);
IR (KBr): ν = 1697 (s, C=O), 1654 (s, C=O) cm⁻¹;
UV/VIS (CHCl₃): λₘₐₓ (ε) = 404 (9447), 512 (34785), 547 (52117) nm.

### d) Herstellung von 1,7-diaroxysubstituierten Perylen-3,4,9,10-tetracarbonsäuredianhydriden II

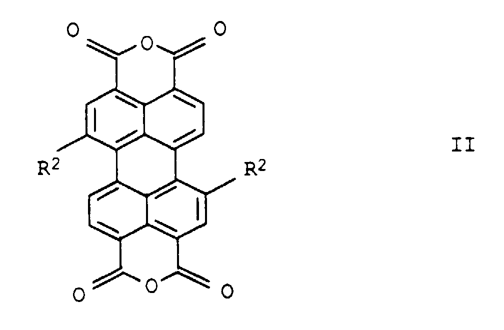

### Beispiele 5 und 6

Eine Mischung von jeweils 10 g des N,N'-Dicyclohexyl-1,7-diaroxyperylen-3,4,9,10-tetracarbonsäurediimids (IV) aus Beispiel 3 bzw. 4, 1 l Isopropanol, 65 g Kaliumhydroxid und 26 g Wasser wurde 5 h unter Rückfluß erhitzt.

Nach dem Abkühlen auf Raumtemperatur wurde das ausgefallene Reaktionsprodukt abfiltriert, mit Isopropanol bis zum farblosen Ablauf gewaschen, dann unter Rühren in 1 l 10 gew.-%ige Salzsäure eingetragen und kurz zum Sieden erhitzt. Nach Abkühlen auf Raumtemperatur wurde das Produkt erneut abfiltriert, mit Wasser neutral gewaschen und bei 100°C im Vakuum getrocknet.

Weitere Einzelheiten zu diesen Versuchen sowie deren Ergebnisse sind in Tabelle 2 zusammengestellt. Die Bestimmung des Wertgehaltes der Produkte erfolgte durch UV/VIS-Spektroskopie und halbquantitative Dünnschichtchromatographie an Kieselgel mit Trichloressigsäure/Toluol als mobile Phase).

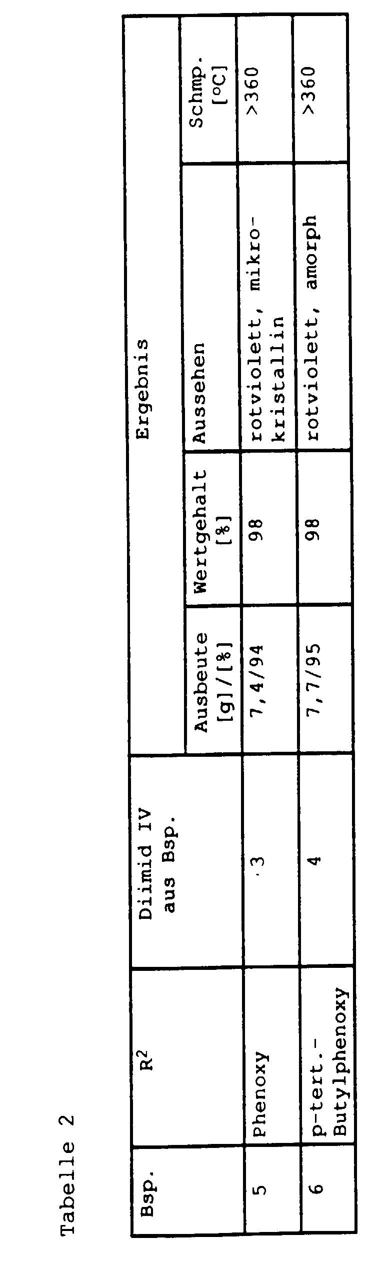

Analytische Daten zu Beispiel 5:
Elementaranalyse (Gew.-% ber./gef.):
C: 75,0/74,8; H: 2,8/2,8; O: 22,2/22,3;
IR (KBr): ν = 1758 (s, C=O), 1729 (s, C=O) cm⁻¹;
UV/VIS (H₂SO₄): λₘₐₓ (ε) = 415 (8832), 559 (38103) nm.

Analytische Daten zu Beispiel 6:
Elementaranalyse (Gew.-% ber./gef.):
C: 76,7/76,6; H: 4,7/4,7; O: 18,6/18,7;
IR (KBr): ν = 1755 (s, C=O), 1730 (s, C=O);
UV/VIS (H₂SO₄): λₘₐₓ (ε) = 412 (9405), 561 (32746) nm.

### Beispiele 7 bis 27

### 1) Herstellung unter Normaldruck (Verfahrensweise V1)

Eine Suspension von 50 mmol Perylen-3,4,9,10-tetracarbonsäuredianhydrid II und x g des Katalysators K in 100 ml Chinolin wurde unter Stickstoff und unter Rühren auf T°C erhitzt. Dann wurde eine Lösung von 50 mmol (Beispiel 22: 55 mmol; Beispiel 23: 52 mmol) des primären Amins R¹NH₂ in 80-100 ml Chinolin in 3-3,5 h (Beispiel 7, 8 und 24: 6 h) zugetropft.

Nach einer Nachrührzeit von t h bei T°C wurde auf etwa 130°C abgekühlt und über eine vorgeheizte G4-Glasfritte filtriert. Nach Abkühlen des Filtrats auf Raumtemperatur wurde das ausgefallene Rohprodukt abfiltriert, mit Methanol gewaschen und unter vermindertem Druck getrocknet. Durch vorsichtige Zugabe von Methanol zum Filtrat wurde die Fällung vervollständigt und das ausgefällte, stärker verunreinigte Rohprodukt wie oben isoliert und mit der Hauptmenge vereinigt.

In Beispiel 23 wurde das bei 130-140°C bereits ausgefallene Rohprodukt abfiltriert, zunächst mit wenig 130°C heißem Chinolin und dann mit Methanol gewaschen und anschließend 0,5 h in 500 ml 10 gew.-%iger Salzsäure unter Rühren auf 80°C erhitzt. Nach Filtration der heißen Suspension wurde das Produkt mit Wasser neutral und salzfrei gewaschen und ebenfalls unter vermindertem Druck getrocknet.

### 2) Herstellung unter Druck (Verfahrensweise V2)

In einem 11-Rührautoklaven wurde eine Mischung aus 0,2 mol (78,4 g) Perylen-3,4,9,10-tetracarbonsäuredianhydrid, x g des Katalysators K, 0,6 mol des primären Amins R¹NH₂ und 400 ml Chinolin 15 min mit Stickstoff gespült. Nach druckdichtem Verschließen des Autoklaven wurde ein Druck von 2 bar Stickstoff voreingestellt, dann wurde auf T°C erhitzt.

Nach einer Nachrührzeit von t h bei T°C und einem Überdruck von maximal 3 bar wurde auf etwa 130°C abgekühlt, entspannt und wie bei der Verfahrensweise V1 allgemein beschrieben aufgearbeitet.

Weitere Einzelheiten zu diesen Versuchen sowie deren Ergebnisse sind in Tabelle 3 zusammengestellt.

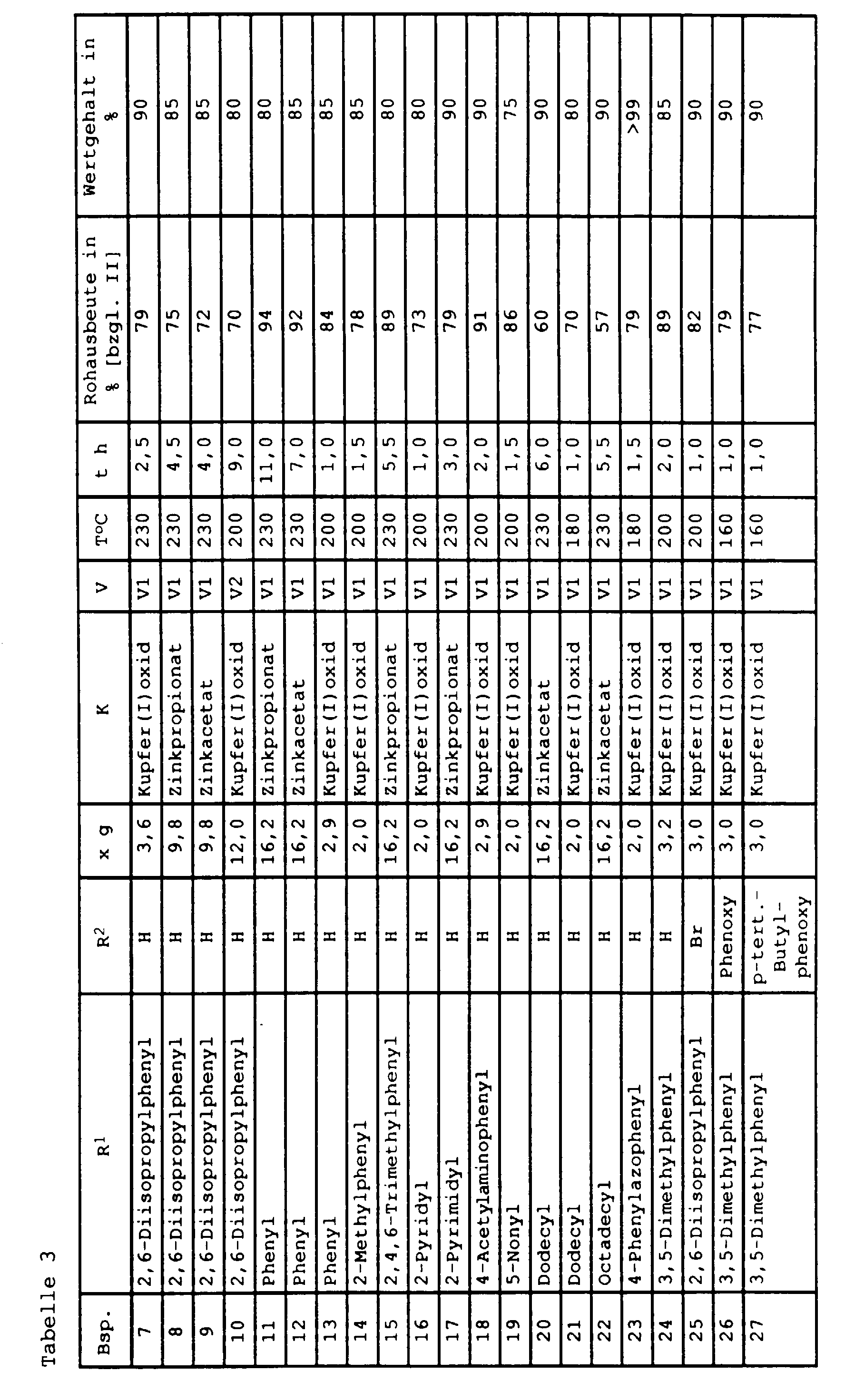

Im folgenden sind analytische Daten für die Imide aus den Beispielen 23, 24 und 26 aufgeführt.

### Beispiel 23:

N-(4-Phenylazophenyl)perylen-3,4-dicarbonsäureimid: rotes Pulver vom Schmp. > 360°C;
Elementaranalyse (Gew.-% ber./gef.):
C: 81,4/80,8; H: 3,8/3,7; N: 8,4/8,3; O: 6,4/6,9;
Masse (EI): m/z = 501 (M⁺), 396 (M⁺ - PhN₂; 100 %);
IR (KBr): ν = 1688 (s, C=O), 1650 (s, C=O) cm⁻¹;
UV/VIS (H₂SO₄): λₘₐₓ (ε) = 413 (27691), 577 (27227), 614 (140738) nm.

### Beispiel 24:

N-(3,5-Dimethylphenyl)perylen-3,4-dicarbonsäureimid: orangerote Kristalle vom Schmp. >360°C;
Elementaranalyse (Gew.-% ber./gef.):
C: 84,7/84,5; H: 4,5/4,5; N: 3,3/3,3; O: 7,5/7,6;
Masse (EI): m/z = 425 (M⁺; 100 %);
IR (KBr): ν = 1695 (s, C=O), 1653 (s, C=O) cm⁻¹;
UV/VIS (CHCl₃): λₘₐₓ (ε) = 265 (29378), 487 (30930), 510 (29375) nm;
1H-NMR (300 MHz, CDCl₃): δ = 8,57 (d,2H); 8,47 (t,4H); 7,94 (d,2H); 7,71-7,64 (dd,2H); 7,15 (s, mit Kernkopplung, 1H); 6,94 (s,2H) ppm.

### Beispiel 26:

N-(3,5-Dimethylphenyl)-1,7-diphenoxyperylen-3,4-dicarbonsäureimid:
schwarzviolette Kristalle vom Schmp. 125-127°C;
Elementaranalyse (Gew.-% ber./gef.):
C: 82,7/82,5: H: 4,5/4,5; N: 2,3/2,3; O: 10,5/10,6;
Masse (EI): m/z = 609 (M⁺; 100 %), 517 (M⁺ - OPh; 58 %); IR (KBr): ν = 1703 (s, C=O), 1670 (s, C=O) cm⁻¹;
UV/VIS (NMP): λₘₐₓ (ε) = 361 (7863), 516 (52443), 742 (722) nm.

### B) Reinigung von Perylen-3,4-dicarbonsäureimiden der Formel Ib

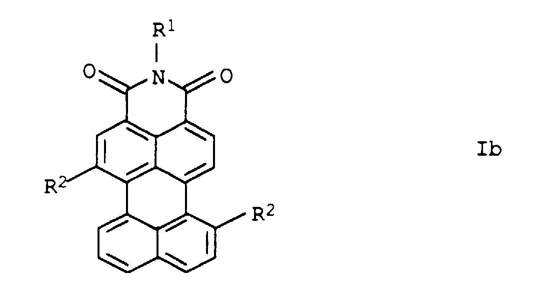

### Beispiele 28 bis 33

Eine Mischung von x g des Rohprodukts aus Beispiel 7 (12; 14; 16; 22; 25) und 100 g N-Methylpyrrolidon (NMP) wurde unter Rühren 15 min auf 165°C erhitzt, dann unter langsamem Rühren auf 50-55°C und anschließend ohne Rühren auf Raumtemperatur abgekühlt. Das ausgefallene NMP-Addukt wurde abfiltriert, nacheinander mit 28 g NMP, 22 g Ethanol und 68 g 6 gew.-%iger Salzsäure gewaschen, dann in Wasser aufgerührt, erneut abfiltriert, mit Wasser neutral gewaschen und unter vermindertem Druck getrocknet.

Das getrocknete NMP-Addukt wurde zerkleinert und in einer Mischung aus 1500 ml Isopropanol, 90,5 g Kaliumhydroxid (85 %ig) und 35 ml Wasser t h unter Rückfluß (ca. 82°C) erhitzt. Nach Abkühlen auf 25°C wurde der Niederschlag abfiltriert, nacheinander mit Isopropanol, wenig Methanol und Wasser gewaschen, anschließend in 100 ml 5 gew.-%iger Salzsäure aufgerührt, erneut abfiltriert, mit Wasser neutral gewaschen und unter vermindertem Druck getrocknet.

Weitere Einzelheiten zu diesen Versuchen sowie deren Ergebnisse sind in Tabelle 4 aufgeführt.

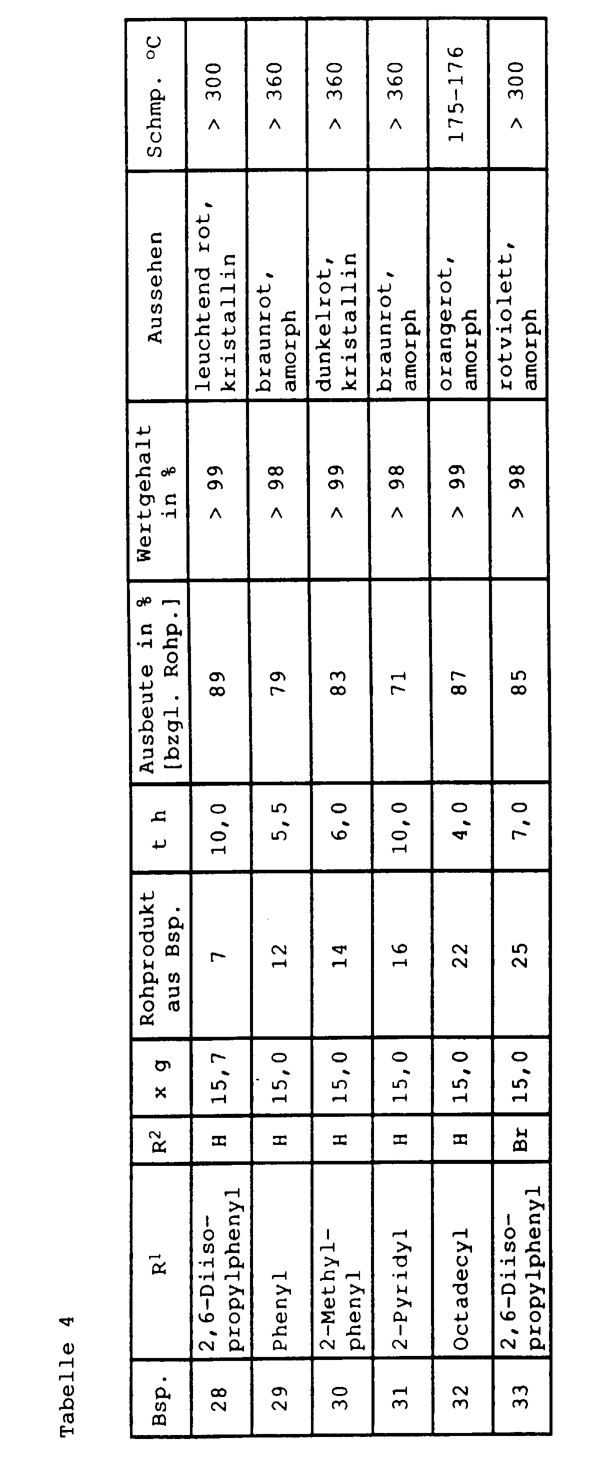

Im folgenden sind analytische Daten für die gereinigten Imide aufgeführt.

### Beispiel 28:

N-(2,6-Diisopropylphenyl)perylen-3,4-dicarbonsäureimid:
¹H-NMR (300 MHz, CDCl₃): δ = 8,60 (d,2H); 8,37 (d,2H); 8,35 (d,2H); 7,85 (d,2H); 7,58 (t,2H); 7,4-7,5 (dd,lH);
7,33 (d,2H); 2,77 (m,2H); 1,18 (d,12H) ppm;
¹³C-NMR (75,5 MHz, CDCl₃): δ = 164,0; 145,7; 137,5; 134,3;
131,9; 131,1; 130,9; 130,5; 129,4; 129,2; 127,9; 127,0;
124,0; 123,8; 121,0; 120,1; 29,1; 24,0 ppm;
IR (KBr): ν = 1696 (s, C=O), 1656 (s, C=O) cm⁻¹;
UV/VIS (CH₂Cl₂): λₘₐₓ (ε) = 263 (33565), 484 (33241), 506 (32061) nm.

### Beispiel 29:

N-Phenylperylen-3,4-dicarbonsäureimid:
Masse (EI): m/z = 397 (M⁺; 100 %)
IR (KBr): ν = 1698 (s, C=O), 1651 (s, C=O) cm⁻¹;
UV/VIS (NMP): λₘₐₓ (ε) = 354 (2890), 498 (29638) nm.

### Beispiel 30:

N-(2-Methylphenyl)perylen-3,4-dicarbonsäureimid:
Masse (EI): m/z = 411 (M⁺), 394 (M⁺ + H - H₂O; 100 %);
IR (KBr): ν = 1682 (s, C=O), 1652 (s, C=O) cm⁻¹;
UV/VIS (NMP): λₘₐₓ (ε) = 355 (3223), 503 (32014) nm.

### Beispiel 31:

N-(2-Pyridyl)perylen-3,4-dicarbonsäureimid:
Masse (FD): m/z = 398 (M⁺; 100 %);
IR (KBr): ν = 1700 (s, C=O), 1654 (s, C=O) cm⁻¹;
UV/VIS (NMP): λₘₐₓ (ε) = 352 (2541), 501 (30328) nm.

### Beispiel 32:

N-Octadecylperylen-3,4-dicarbonsäureimid:
¹H-NMR (250 MHz, CD₂Cl₂):
δ = 8,37 (d,2H); 8,25 (d,2H); 8,19 (d,2H); 7,77 (d,2H);
7,50 (t,2H); 4,05 (t,2H); 1,15-1,42 (m,32H); 0,81 (t,3H) ppm;
Masse (FAB): m/z = 573 (M⁺), 321 (M⁺ - C₁₈H₂₅; 100 %);
IR (KBr): ν = 1680 (m, C-O), 1649 (s, C=O) cm⁻¹;
UV/VIS (H₂SO₄): λₘₐₓ (ε) = 410 (6158), 574 (21297), 613 (168942) nm.

### Beispiel 33:

N-(2,6-Diisopropylphenyl)-1,7-dibromperylen-3,4-dicarbonsäureimid:
Masse (EI): m/z = 642/640/638(M⁺, ⁷⁹Br/⁸¹Br),
560/558 (M⁺ - Br; 100 %);
IR (KBr): ν = 1695 (s, C=O), 1656 (s, C=O) cm⁻¹;
UV/VIS (H₂SO₄): λₘₐₓ (ε) = 399 (13521), 548 (41551) nm.

## Patentansprüche (Patentansprüche für folgende(n) Vertragsstaat(en): AT, BE, DK, ES, GR, IE, IT, LU, MC, NL, PT, SE)

1. Verfahren zur Herstellung von Perylen-3,4-dicarbonsäureimiden durch Umsetzung einer Perylen-3,4,9,10-tetracarbonsäure oder der entsprechenden Anhydride mit einem primären Amin, dadurch gekennzeichnet, daß man die Umsetzung in Gegenwart einer tertiären stickstoffbasischen Verbindung als Lösungsmittel und eines Übergangsmetalls oder Übergangsmetallsalzes als Katalysator vornimmt.

2. Verfahren nach Anspruch 1, dadurch gekennzeichnet, daß man als tertiäre stickstoffbasische Verbindung einen stickstoffhaltigen Heneroaromaten verwenden.

3. Verfahren nach Anspruch 1 oder 2, dadurch gekennzeichnet, daß man als Katalysator Zink, Zinksalze, Kupfer, Kupfersalze oder Gemische davon verwendet.

4. Verfahren zur Reinigung von Perylen-3,4-dicarbonsäureimiden, die durch Umsetzung einer Perylen-3,4,9,10-tetracarbonsäure oder der entsprechenden Anhydride mit einem primären Amin erhalten worden sind, dadurch gekennzeichnet, daß man die Rohprodukte zunächst in N-Methylpyrrolidon erhitzt und die gebildeten N-Methylpyrrolidon-Addukte dann in Gegenwart eines organischen Verdünnungsmittels mit einer Base behandelt und die danach isolierten Produkte gewünschtenfalls einer zusätzlichen Behandlung mit einer wäßrigen Säure unterzieht.

5. Verfahren nach Anspruch 4, dadurch gekennzeichnet, daß man als Base ein Alkalimetallhydroxid oder ein Alkalimetallalkoholat verwendet.

6. Verfahren zur Reindarstellung von Perylen-3,4-dicarbonsäureimiden durch Umsetzung einer Perylen-3,4,9,10-tetracarbonsäure oder der entsprechenden Anhydride mit einem primären Amin, dadurch gekennzeichnet, daß man die Umsetzung in Gegenwart einer tertiären stickstoffbasischen Verbindung als Lösungsmittel und eines Übergangsmetalls oder Übergangsmetallsalzes als Katalysator vornimmt und die erhaltenen Rohprodukte zunächst in N-Methylpyrrolidon erhitzt und die gebildeten N-Methylpyrrolidon-Addukte dann in Gegenwart eines organischen Verdünnungsmittels mit einer Base behandelt und die danach isolierten Produkte gewünschtenfalls einer zusatzlichen Behandlung mit einer wäßrigen Säure unterzieht.

7. Perylen-3,4-dicarbonsäureimide der allgemeinen Formel Ia 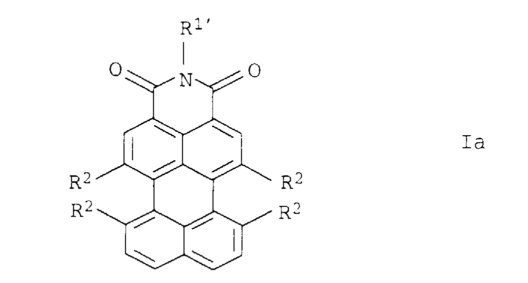 in der die Variablen folgende Bedeutung haben:
R^{1'} C₁₄-C₃₀-Alkyl, dessen Kohlenstoffkette durch eine oder mehrere Gruppierungen -O-, -S-, -NR³-, -CO- und/oder -SO₂- unterbrochen sein kann und das durch Carboxy, Sulfo, Hydroxy, Cyano, C₁-C₆-Alkoxy oder einen über ein Stickstoffatom gebundenen 5- bis 7-gliedrigen heterocyclischen Rest, der weitere Heteroatome enthalten und aromatisch sein kann, ein- oder mehrfach substituiert sein kann, wobei
R³ Wasserstoff oder C₁-C₆-Alkyl bedeutet;
C₅-C₈-Cycloalkyl, dessen Kohlenstoffgerüst durch eine oder mehrere Gruppierungen -O-, -S- und/oder -NR³- unterbrochen sein kann;
Phenyl, das durch C₁-C₄-Alkyl oder Methoxy mindestens in beiden ortho-Positionen, durch C₅-C₁₈-Alkyl, C₂-C₆-Alkoxy, Halogen, Hydroxy, Cyano, Carboxy, -CONHR⁴, -NHCOR⁴ und/oder Aryl- oder Hetarylazo, das jeweils durch
C₁-C₁₀-Alkyl, C₁-C₆-Alkoxy, Halogen, Hydroxy, Cyano oder Carboxy substituiert sein kann, ein- oder mehrfach substituiert ist, wobei
R⁴ Wasserstoff; C₁-C₁₈-Alkyl; Aryl oder Hetaryl, das jeweils durch C₁-C₆-Alkyl, C₁-C₆-Alkoxy, Halogen, Hydroxy oder Cyano substituiert sein kann, bedeutet;
Naphthyl oder Hetaryl, das jeweils durch die für Phenyl genannten Substituenten substituiert sein kann, wobei die C₁-C₄-Alkyl- und C₁-C₆-Alkoxysubstituenten beliebige Positionen am Ringsystem einnehmen können;
R² unabhängig voneinander Wasserstoff; Halogen; C₁-C₁₈-Alkyl; Aryloxy, Arylthio, Hetaryloxy oder Hetarylthlo, das jeweils durch C₁-C₁₀-Alkyl, C₁-C₆-Alkoxy, Cyano oder Carboxy substituiert sein kann.

8. Perylen-3,4-dicarbonsäureimide der Formel Ia nach Anspruch 7, in der die Variablen folgende Bedeutung haben:
R^{1'} C₁₄-C₃₀-Alkyl, das durch Carboxy, Sulfo, Hydroxy oder einen über ein Stickstoffatom gebundenen 5- bis 7-gliedrigen heterocyclischen Rest, der weitere Heteroatome enthalten und aromatisch sein kann, einfach substituiert ist; C₅-C₈-Cycloalkyl;
Phenyl, das durch C₁-C₄-Alkyl mindestens in beiden ortho-Positionen, Halogen, Hydroxy, Cyano, Carboxy, -COHNR⁴ oder -NHCOR⁴ ein- oder mehrfach und/oder durch Aryl- oder Hetarylazo, das jeweils durch C₁-C₁₀-Alkyl, C₁-C₆-Alkoxy, Halogen, Hydroxy, Cyano oder Carboxy substituiert sein kann, einfach substituiert ist;
Hetaryl, das durch die für Phenyl genannten Substituenten und/oder C₁-C₄-Alkoxy substituiert sein kann, wobei die C₁-C₄-Alkylsubstituenten beliebige Positionen am Ringsystem einnehmen können;
R² unabhängig voneinander Wasserstoff, Halogen oder Phenoxy, das durch C₁-C₆-Alkyl, C₁-C₆-oxy, Cyano oder Carboxy substituiert sein kann.

9. Perylen-3,4-dicarbonsäureimide der Formel Ia nach Anspruch 7, in der die Variablen folgende Bedeutung haben:
R^{1'} C₁₄-C₃₀-Alkyl, das durch Carboxy, Sulfo oder Hydroxy einfach substituiert ist;
C₅-C₇-Cycloalkyl;
Phenyl, das durch C₁-C₄-Alkyl in beiden ortho-Positionen, Hydroxy, Cyano, Carboxy, -CONHR⁴ oder -NHCOR⁴, wobei
R⁴ C₁-C₄-Alkyl oder Phenyl, das durch C₁-C₄-Alkyl, C₁-C₄-Alkoxy oder Cyano substituiert sein kann, bedeutet,
oder durch Phenyl- oder Naphthylazo, das durch C₁-C₄-Alkyl, C₁-C₄-Alkoxy, Hydroxy oder Cyano substituiert sein kann, einfach substituiert ist;
monocyclisches Hetaryl, das durch C₁-C₄-Alkyl, C₁-C₄-Alkoxy, Hydroxy oder Cyano ein- oder mehrfach oder durch Phenyl- oder Naphthylazo, das durch C₁-C₄-Alkyl, C₁-C₄-Alkoxy, Hydroxy oder Cyano substituiert sein kann, einfach substituiert sein kann;
R² unabhängig voneinander Wasserstoff oder Phenoxy, das durch C₁-C₄-Alkyl substituiert sein kann.

10. Verwendung von Perylen-3,4-dicarbonsäureimiden der Formel Ia gemäß den Ansprüchen 7 bis 9 als Fluoreszenzfarbstoffe, Pigmente oder Pigmentadditivvorstufen.

## Patentansprüche (Patentansprüche für folgende(n) Vertragsstaat(en): CH, DE, FR, GB, LI)

1. Verfahren zur Herstellung von Perylen-3,4-dicarbonsäureimiden durch Umsetzung einer Perylen-3,4,9,10-tetracarbonsäure oder der entsprechenden Anhydride mit einem primäre Amin, dadurch gekennzeichnet, daß man die Umsetzung in einem im wesentlichen wasserfreien Reaktionsmedium in Gegenwart einer tertiären stickstoffbasischen Verbindung als Lösungsmittel und eines Übergangsmetalls oder Übergangsmetallsalzes als Katalysator vornimmt.

2. Verfahren nach Anspruch 1, dadurch gekennzeichnet, daß man als tertiäre stickstoffbasische Verbindung einen stickstoffhaltigen Heteroaromaten verwendet.

3. Verfahren nach Anspruch 1 oder 2, dadurch gekennzeichnet, daß man als Katalysator Zink, Zinksalze, Kupfer, Kupfersalze oder Gemische davon verwendet.

4. Verfahren zur Reinigung von Perylen-3,4-dicarbonsäureimiden, die durch Umsetzung einer Perylen-3,4,9,10-tetracarbonsäure oder der entsprechenden Anhydride mit einem primären Amin erhalten worden sind, dadurch gekennzeichnet, daß man die Rohprodukte zunächst in N-Methylpyrrolidon erhitzt und die gebildeten N-Methylpyrrolidon-Addukte dann in Gegenwart eines organischen Verdünnungsmittels mit einer Base behandelt und die danach isolierten Produkte gewünschtenfalls einer zusätzlichen Behandlung mit einer wäßrigen Säure unterzieht.

5. Verfahren nach Anspruch 4, dadurch gekennzeichnet, daß man als Base ein Alkalimetallhydroxid oder ein Alkalimetallalkoholat verwenden.

6. Verfahren zur Reindarstellung von Perylen-3,4-dicarbonsäureimiden durch Umsetzung einer Perylen-3,4,9,10-tetracarbonsäure oder der entsprechenden Anhydride mit einem primären Amin, dadurch gekennzeichnet, daß man die Umsetzung in Gegenwart einer tertiären stickstoffbasischen Verbindung als Lösungsmittel und eines Übergangsmetalls oder Übergangsmetallsalzes als Katalysator vornimmt und die erhaltenen Rohprodukte zunächst in N-Methylpyrrolidon erhitzt und die gebildeten N-Methylpyrrolidon-Addukte dann in Gegenwart eines organischen Verdünnungsmittels mit einer Base behandelt und die danach isolierten Produkte gewünschtenfalls einer zusätzlichen Behandlung mit einer wäßrigen Säure unterzieht.

7. Perylen-3,4-dicarbonsäureimide der allgemeinen Formel Ia 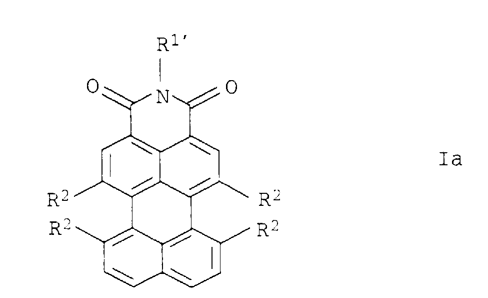 in der die Variablen folgende Bedeutung haben:
R^{1'} C₁₄-C₃₀-Alkyl, dessen Kohlenstoffkette durch eine oder mehrere Gruppierungen -O-, -S-, -NR³-, -CO- und/oder -SO₂- unterbrochen sein kann und das durch Carboxy, Sulfo, Hydroxy, Cyano, C₁-C₆-Alkoxy oder einen über ein Stickstoffatom gebundenen 5- bis 7-gliedrigen heterocyclischen Rest, der weitere Heteroatome enthalten und aromatisch sein kann, ein- oder mehrfach substituiert sein kann, wobei
R³ Wasserstoff oder C₁-C₆-Alkyl bedeutet;
C₅-C₈-Cycloalkyl, dessen Kohlenstoffgerüst durch eine oder mehrere Gruppierungen -O-, -S- und/oder -NR³- unterbrochen sein kann;
Phenyl, das durch C₁-C₄-Alkyl oder Methoxy mindestens in beiden ortho-Positionen, durch C₅-C₁₈-Alkyl, C₂-C₆-Alkoxy, Halogen, Hydroxy, Cyano, Carboxy, -CONHR⁴, -NHCOR⁴ und/oder Aryl- oder Hetarylazo, das jeweils durch C₁-C₁₀-Alkyl, C₁-C₆-Alkoxy, Halogen, Hydroxy, Cyano oder Carboxy substituiert sein kann, ein- oder mehrfach substituiert ist, wobei
R⁴ Wasserstoff; C₁-C₁₈-Alkyl; Aryl oder Hetaryl, das jeweils durch C₁-C₆-Alkyl, C₁-C₆-Alkoxy, Halogen, Hydroxy oder Cyano substituiert sein kann, bedeutet;
Naphthyl oder Hetaryl, das jeweils durch die für Phenyl genannten Substituenten substituiert sein kann, wobei die C₁-C₄-Alkyl- und C₁-C₆-Alkoxysubstituenten beliebige Positionen am Ringsystem einnehmen können;
R² unabhängig voneinander Wasserstoff; Halogen; C₁-C₁₈-Alkyl; Aryloxy, Arylthio, Hetaryloxy oder Hetarylthio, das jeweils durch C₁-C₁₀-Alkyl, C₁-C₆-Alkoxy, Cyano oder Carboxy substituiert sein kann,
wobei N-(1-Tetradecyl)-, -(1-Octylnonyl)-, -(1-Nonyldecyl)- und- (C₅-C₈-Cycloalkyl)perylen-3,4-dicarbonsäureimid ausgeschlossen sind.

8. Perylen-3,4-dicarbonsäureimide der Formel Ia nach Anspruch 7, in der die Variablen folgende Bedeutung haben:
R^{1'} C₁₄-C₃₀-Alkyl, das durch Carboxy, Sulfo, Hydroxy oder einen über ein Stickstoffatom gebundenen 5- bis 7-gliedrigen heterocyclischen Rest, der weitere Heteroatome enthalten und aromatisch sein kann, einfach substituiert ist;
C₅-C₈-Cycloalkyl; Phenyl, das durch C₁-C₄-Alkyl mindestens in beiden ortho-Positionen, Halogen, Hydroxy, Cyano, Carboxy, -COHNR⁴ oder -NHCOR⁴ ein- oder mehrfach und/oder durch Aryl- oder Hetarylazo, das jeweils durch C₁-C₁₀-Alkyl, C₁-C₆-Alkoxy, Halogen, Hydroxy, Cyano oder Carboxy substituiert sein kann, einfach substituiert ist;
Hetaryl, das durch die für Phenyl genannten Substituenten und/oder C₁-C₄-Alkoxy substituiert sein kann, wobei die C₁-C₄-Alkylsubstituenten beliebige Positionen am Ringsystem einnehmen können;
R² unabhängig voneinander Wasserstoff, Halogen oder Phenoxy, das durch C₁-C₆-Alkyl, C₁-C₆-Alkoxy, Cyano oder Carboxy substituiert sein kann,
wobei N-Cyclopentyl-, -Cyclohexyl-, -Cycloheptyl- und -Cyclooctylperylen-3,4-dicarbonsäureimid ausgeschlossen sind.

9. Perylen-3,4-dicarbonsäureimide der Formel Ia nach Anspruch 7, in der die Variablen folgende Bedeutung haben:
R^{1'} C₁₄-C₃₀-Alkyl, das durch Carboxy, Sulfo oder Hydroxy einfach substituiert ist;
C₅-C₇-Cycloalkyl;
Phenyl, das durch C₁-C₄-Alkyl in beiden ortho-Positionen, Hydroxy, Cyano, Carboxy, -CONHR⁴ oder -NHCOR⁴, wobei
R⁴ C₁-C₄-Alkyl oder Phenyl, das durch C₁-C₄-Alkyl, C₁-C₄-Alkoxy oder Cyano substituiert sein kann, bedeutet,
oder durch Phenyl- oder Naphthylazo, das durch C₁-C₄-Alkyl, C₁-C₄-Alkoxy, Hydroxy oder Cyano substituiert sein kann, einfach substituiert ist;
monocyclisches Hetaryl, das durch C₁-C₄-Alkyl, C₁-C₄-Alkoxy, Hydroxy oder Cyano ein- oder mehrfach oder durch Phenyl- oder Naphthylazo, das durch C₁-C₄-Alkyl, C₁-C₄-Alkoxy, Hydroxy oder Cyano substituiert sein kann, einfach substituiert sein kann;
R² unabhängig voneinander Wasserstoff oder Phenoxy, das durch C₁-C₄-Alkyl substituiert sein kann,
wobei N-Cyclopentyl-, -Cyclohexyl- und -Cycloheptylperylen-3,4-dicarbonsäureimid ausgeschlossen sind.

10. Verwendung von Perylen-3,4-dicarbonsäureimiden der Formel Ia gemäß den Ansprüchen 7 bis 9 als Fluoreszenzfarbstoffe, Pigmente oder Pigmentadditivvorstufen.

## Claims (Claims for the following Contracting State(s): AT, BE, DK, ES, GR, IE, IT, LU, MC, NL, PT, SE)

1. A process for preparing perylene-3,4-dicarbimides by reacting a perylene-3,4,9,10-tetracarboxylic acid or the corresponding anhydrides with a primary amine, which comprises performing the reaction in the presence of a tertiary nitrogen base as solvent and of a transition metal or transition metal salt as catalyst.

2. A process as claimed in claim 1, wherein the tertiary nitrogen base used is a nitrogen-containing heteroaromatic.

3. A process as claimed in claim 1 or 2, wherein the catalyst used is zinc, a zinc salt, copper, a copper salt or a mixture thereof.

4. A process for purifying perylene-3,4-dicarbimides obtained by reaction of a perylene-3,4,9,10-tetracarboxylic acid or of the corresponding anhydrides with a primary amine, which comprises first heating the crude products in N-methylpyrrolidone, then treating the resulting N-methylpyrrolidone adducts with a base in the presence of an organic diluent, and, if desired, subjecting the subsequently isolated products to an additional treatment with an aqueous acid.

5. A process as claimed in claim 4, wherein the base used is an alkali metal hydroxide or an alkali metal alkoxide.

6. A process for making pure perylene-3,4-dicarbimides by reacting a perylene-3,4,9,10-tetracarboxylic acid or the corresponding anhydrides with a primary amine, which comprises performing the reaction in the presence of a tertiary nitrogen base as solvent and of a transition metal or transition metal salt as catalyst and first heating the crude products in N-methylpyrrolidone, then treating the resulting N-methylpyrrolidone adducts with a base in the presence of an organic diluent, and, if desired, subjecting the subsequently isolated products to an additional treatment with an aqueous acid.

7. Perylene-3,4-dicarbimides of the general formula Ia 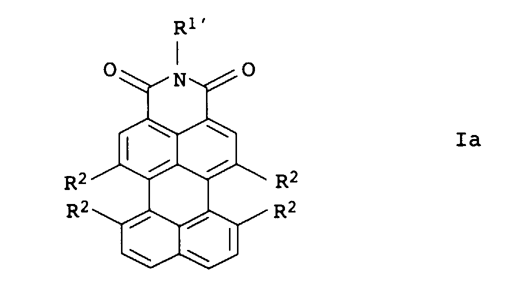 where
R^{1'} is C₁₄-C₃₀-alkyl whose carbon chain may be interrupted by one or more of -O-, -S-, -NR³-, -CO- and/or -SO₂- and which may be monosubstituted or polysubstituted by carboxyl, sulfo, hydroxyl, cyano, C₁-C₆-alkoxy or a 5-, 6- or 7-membered heterocyclic radical which is attached via a nitrogen atom and which may contain further heteroatoms and may be aromatic, where
R³ is hydrogen or C₁-C₆-alkyl;
C₅-C₈-cycloalkyl whose carbon skeleton may be interrupted by one or more of -O-, -S- and/or -NR³-;
phenyl which is monosubstituted or polysubstituted by C₁-C₄-alkyl or methoxy at least in the two ortho positions, by C₅-C₁₈-alkyl, C₂-C₆-alkoxy, halogen,
hydroxyl, cyano, carboxyl, -CONHR⁴, -NHCOR⁴ and/or aryl- or hetaryl-azo, which may each be substituted by C₁-C₁₀-alkyl, C₁-C₆-alkoxy, halogen, hydroxyl, cyano or carboxyl, where
R⁴ is hydrogen; C₁-C₁₈-alkyl; aryl or hetaryl, which may each be substituted by C₁-C₆-alkyl, C₁-C₆-alkoxy, halogen, hydroxyl or cyano;
naphthyl or hetaryl, which may each be substituted by the substituents mentioned for phenyl, in which case the C₁-C₄-alkyl and C₁-C₆-alkoxy substituents may be in any desired position of the ring system;
R² is in each instance independently of the other instances hydrogen; halogen; C₁-C₁₈-alkyl; aryloxy, arylthio, hetaryloxy or hetarylthio, which may each be substituted by C₁-C₁₀-alkyl, C₁-C₆-alkoxy, cyano or carboxyl.

8. Perylene-3,4-dicarbimides as claimed in claim 7 of the formula Ia where
R^{1'} is C₁₄-C₃₀-alkyl which is monosubstituted by carboxyl, sulfo, hydroxyl or a 5-, 6- or 7-membered heterocyclic radical which is attached by a nitrogen atom and which may contain further heteroatoms and may be aromatic;
C₅-C₈-cycloalkyl;
phenyl which is mono or polysubstituted by C₁-C₄-alkyl at least in the two ortho positions, halogen, hydroxyl, cyano, carboxyl, -COHNR⁴ or -NHCOR⁴ and/or monosubstituted by aryl- or hetaryl-azo, which may each be substituted by C₁-C₁₀-alkyl, C₁-C₆-alkoxy, halogen, hydroxyl, cyano or carboxyl;
hetaryl which may be substituted by the substituents mentioned for phenyl and/or by C₁-C₄-alkoxy, in which case the C₁-C₄-alkyl substituents may be in any desired position on the ring system;
R² is in each instance independently of the other instances hydrogen, halogen or phenoxy which may be substituted by C₁-C₆-alkyl, C₁-C₆-alkoxy, cyano or carboxyl.

9. Perylene-3,4-dicarbimides as claimed in claim 7 of the formula Ia where
R^{1'} is C₁₄-C₃₀-alkyl which is monosubstituted by carboxyl, sulfo or hydroxyl;
C₅-C₇-cycloalkyl;
phenyl which is monosubstituted by C₁-C₄-alkyl in both ortho positions, hydroxyl, cyano, carboxyl, -CONHR⁴ or -NHCOR⁴, where
R⁴ is C₁-C₄-alkyl or phenyl which may be substituted by C₁-C₄-alkyl, C₁-C₄-alkoxy or cyano,
or by phenyl- or naphthyl-azo which may be substituted by C₁-C₄-alkyl, C₁-C₄-alkoxy, hydroxyl or cyano;
monocyclic hetaryl which may be monosubstituted or polysubstituted by C₁-C₄-alkyl, C₁-C₄-alkoxy, hydroxyl or cyano or monosubstituted by phenyl- or naphthyl-azo which may be substituted by C₁-C₄-alkyl, C₁-C₄-alkoxy, hydroxyl or cyano;
R² is in each instance independently of the other instances hydrogen or phenoxy which may be substituted by C₁-C₄-alkyl.

10. The use of perylene-3,4-dicarbimides of the formula Ia as set forth in claims 7 to 9 as fluorescent dyes, pigments or pigment additive precursors.

## Claims (Claims for the following Contracting State(s): CH, DE, FR, G,B LI)

1. A process for preparing perylene-3,4-dicarbimides by reacting a perylene-3,4,9,10-tetracarboxylic acid or the corresponding anhydrides with a primary amine, which comprises performing the reaction in an essentially anhydrous reaction medium in the presence of a tertiary nitrogen base as solvent and of a transition metal or transition metal salt as catalyst.

2. A process as claimed in claim 1, wherein the tertiary nitrogen base used is a nitrogen-containing heteroaromatic.

3. A process as claimed in claim 1 or 2, wherein the catalyst used is zinc, a zinc salt, copper, a copper salt or a mixture thereof.

4. A process for purifying perylene-3,4-dicarbimides obtained by reaction of a perylene-3,4,9,10-tetracarboxylic acid or of the corresponding anhydrides with a primary amine, which comprises first heating the crude products in N-methylpyrrolidone, then treating the resulting N-methylpyrrolidone adducts with a base in the presence of an organic diluent, and, if desired, subjecting the subsequently isolated products to an additional treatment with an aqueous acid.

5. A process as claimed in claim 4, wherein the base used is an alkali metal hydroxide or an alkali metal alkoxide.

6. A process for making pure perylene-3,4-dicarbimides by reacting a perylene-3,4,9,10-tetracarboxylic acid or the corresponding anhydrides with a primary amine, which comprises performing the reaction in the presence of a tertiary nitrogen base as solvent and of a transition metal or transition metal salt as catalyst and first heating the crude products in N-methylpyrrolidone, then treating the resulting N-methylpyrrolidone adducts with a base in the presence of an organic diluent, and, if desired, subjecting the subsequently isolated products to an additional treatment with an aqueous acid.

7. Perylene-3,4-dicarbimides of the general formula Ia 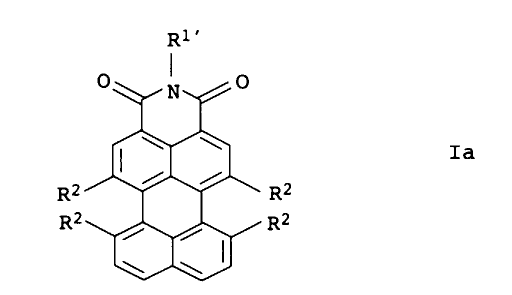 where
R^{1'} is C₁₄-C₃₀-alkyl whose carbon chain may be interrupted by one or more of -O-, -S-, -NR³-, -CO- and/or -SO₂- and which may be monosubstituted or polysubstituted by carboxyl, sulfo, hydroxyl, cyano, C₁-C₆-alkoxy or a 5-, 6- or 7-membered heterocyclic radical which is attached via a nitrogen atom and which may contain further heteroatoms and may be aromatic, where
R³ is hydrogen or C₁-C₆-alkyl;
C₅-C₈-cycloalkyl whose carbon skeleton may be interrupted by one or more of -O-, -S- and/or -NR³-;
phenyl which is monosubstituted or polysubstituted by C₁-C₄-alkyl or methoxy at least in the two ortho positions, by C₅-C₁₈-alkyl, C₂-C₆-alkoxy, halogen, hydroxyl, cyano, carboxyl, -CONHR⁴, -NHCOR⁴ and/or aryl- or hetaryl-azo, which may each be substituted by C₁-C₁₀-alkyl, C₁-C₆-alkoxy, halogen, hydroxyl, cyano or carboxyl, where
R⁴ is hydrogen; C₁-C₁₈-alkyl; aryl or hetaryl, which may each be substituted by C₁-C₆-alkyl, C₁-C₆-alkoxy, halogen, hydroxyl or cyano;
naphthyl or hetaryl, which may each be substituted by the substituents mentioned for phenyl, in which case the C₁-C₄-alkyl and C₁-C₆-alkoxy substituents may be in any desired position of the ring system;
R² is in each instance independently of the other instances hydrogen; halogen; C₁-C₁₈-alkyl; aryloxy, arylthio, hetaryloxy or hetarylthio, which may each be substituted by C₁-C₁₀-alkyl, C₁-C₆-alkoxy, cyano or carboxyl,
excluding N-(1-tetradecyl)-, -(1-octylnonyl)-, -(1-nonyldecyl)- and -(C₅-C₈-cycloalkyl)-perylene-3,4-dicarbimide.

8. Perylene-3,4-dicarbimides as claimed in claim 7 of the formula Ia where
R^{1'} is C₁₄-C₃₀-alkyl which is monosubstituted by carboxyl, sulfo, hydroxyl or a 5-, 6- or 7-membered heterocyclic radical which is attached by a nitrogen atom and which may contain further heteroatoms and may be aromatic;
C₅-C₈-cycloalkyl;
phenyl which is mono or polysubstituted by C₁-C₄-alkyl at least in the two ortho positions, halogen, hydroxyl,
cyano, carboxyl, -COHNR⁴ or -NHCOR⁴ and/or monosubstituted by aryl- or hetaryl-azo, which may each be substituted by C₁-C₁₀-alkyl, C₁-C₆-alkoxy, halogen, hydroxyl, cyano or carboxyl;
hetaryl which may be substituted by the substituents mentioned for phenyl and/or by C₁-C₄-alkoxy, in which case the C₁-C₄-alkyl substituents may be in any desired position on the ring system;
R² is in each instance independently of the other instances hydrogen, halogen or phenoxy which may be substituted by C₁-C₆-alkyl, C₁-C₆-alkoxy, cyano or carboxyl,
excluding N-cyclopentyl-, -cyclohexyl-, -cycloheptyl- and -cyclooctyl-perylene-3,4-dicarbimide.

9. Perylene-3,4-dicarbimides as claimed in claim 7 of the formula Ia where
R^{1'} is C₁₄-C₃₀-alkyl which is monosubstituted by carboxyl, sulfo or hydroxyl;
C₅-C₇-cycloalkyl;
phenyl which is monosubstituted by C₁-C₄-alkyl in both ortho positions, hydroxyl, cyano, carboxyl, -CONHR⁴ or -NHCOR⁴, where
R⁴ is C₁-C₄-alkyl or phenyl which may be substituted by C₁-C₄-alkyl, C₁-C₄-alkoxy or cyano,
or by phenyl- or naphthyl-azo which may be substituted by C₁-C₄-alkyl, C₁-C₄-alkoxy, hydroxyl or cyano;
monocyclic hetaryl which may be monosubstituted or polysubstituted by C₁-C₄-alkyl, C₁-C₄-alkoxy, hydroxyl or cyano or monosubstituted by phenyl- or naphthyl-azo which may be substituted by C₁-C₄-alkyl, C₁-C₄-alkoxy, hydroxyl or cyano;
R² is in each instance independently of the other instances hydrogen or phenoxy which may be substituted by C₁-C₄-alkyl,
excluding N-cyclopentyl-, -cyclohexyl- and -cycloheptyl-perylene-3,4-dicarbimide.

10. The use of perylene-3,4-dicarbimides of the formula Ia as set forth in claims 7 to 9 as fluorescent dyes, pigments or pigment additive precursors.

## Revendications (Revendications pour l'(les) Etat(s) contractant(s) suivant(s): AT, BE, DK, ES, GR, IE, IT, LU, MC, NL, PT, SE)

1. Procédé de préparation d'imides de pérylène-3,4-dicarboxylique par réaction d'un pérylène-3,4,9,10-tétracarboxylique ou de l'anhydride correspondant avec une amine primaire, caractérisé en ce que l'on effectue la réaction en présence d'un composé basique azoté tertiaire en tant que solvant et d'un métal de transition ou d'un sel de métal de transition en tant que catalyseur.

2. Procédé selon la revendication 1, caractérisé en ce que l'on utilise un composé hétéroaromatique contenant de l'azote en tant que composé basique azoté tertiaire.

3. Procédé selon la revendication 1 ou 2, caractérisé en ce que l'on utilise en tant que catalyseur du zinc, des sels de zinc, du cuivre, des sels de cuivre ou des mélanges de ceux-ci.

4. Procédé de purification d'imides de pérylène-3,4-dicarboxylique obtenus par réaction d'un pérylène-3,4,9,10-tétracarboxylique ou de l'anhydride correspondant avec une amine primaire, caractérisé en ce que l'on chauffe tout d'abord les produits bruts dans de la N-méthylpyrrolidone puis en ce que l'on traite ensuite les adduits formés avec la N-méthylpyrrolidone avec une base, en présence d'un diluant organique, et l'on fait éventuellement subir aux produits isolés un traitement supplémentaire avec un acide aqueux.

5. Procédé selon la revendication 4, caractérisé en ce que l'on utilise en tant que base un hydroxyde de métal alcalin ou un alcoolate de métal alcalin.

6. Procédé de purification d'imides de pérylène-3,4-dicarboxylique obtenus par réaction d'un pérylène-3,4,9,10-tétracarboxylique ou de l'anhydride correspondant avec une amine primaire, caractérisé en ce que l'on effectue la réaction en présence d'un composé basique azoté tertiaire en tant que solvant et d'un métal de transition ou d'un sel de métal de transition en tant que catalyseur, et en ce que l'on chauffe tout d'abord les produits bruts dans de la N-méthylpyrrolidone puis en ce que l'on traite ensuite les adduits formés avec la N-méthylpyrrolidone avec une base, en présence d'un diluant organique, et l'on fait éventuellement subir aux produits isolés un traitement supplémentaire avec un acide aqueux.

7. Imides de pérylène-3,4-dicarboxylique de formule générale la 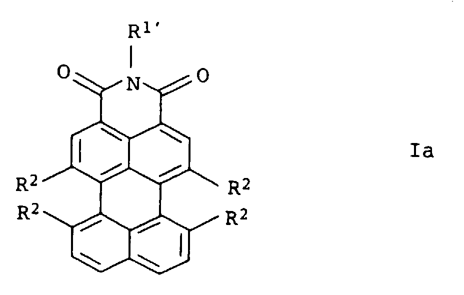 dans laquelle les variables ont la signification suivante :
R^{1'} représente un groupement alkyle en C₁₄-C₃₀, dont la chaîne carbonée peut être interrompue par un ou plusieurs groupements -O-, -S-, -NR³-, -CO- et/ou -SO₂- et pouvant être substitué une ou plusieurs fois par un groupement carboxy, sulfo, hydroxy, cyano, alcoxy en C₁-C₆, ou un reste hétérocyclique à 5-7 maillons relié par un atome d'azote et qui peut contenir d'autres hétéroatomes et être aromatique, où
R³ représente un atome d'hydrogène ou un groupement alkyle en C₁-C₆;
un groupement cycloalkyle en C₅-C₈ dont le squelette carboné peut être interrompu par un ou plusieurs groupements -O-, -S-,
et/ou -NR³-;
un groupement phényle substitué une ou plusieurs fois par un groupement alkyle en C₁-C₄ ou méthoxy dans les deux positions ortho au moins, des groupements alkyle en
C₅-C₁₈, alcoxy en C₂-C₆, des atomes d'halogène, des groupements hydroxy, cyano, carboxy, -CONHR⁴, -NHCOR⁴ et/ou aryl- ou hétarylazo, qui peuvent chacun être substitués par des groupements alkyle en C₁-C₁₀, alcoxy en C₁-C₆, des atomes d'halogène, des groupements hydroxy, cyano ou carboxy, où
R⁴ représente un atome d'hydrogène, un groupement alkyle en C₁-C₁₈, aryle ou hétaryle pouvant chacun être substitué par des groupements alkyle en C₁-C₆, alcoxy en C₁-C₆, des atomes d'halogène, des groupements hydroxy ou cyano ;
un groupement naphtyle ou hétaryle, pouvant chacun être substitué par les substituants cités pour le groupement phényle, où les substituants alkyle en C₁-C₄ et alcoxy en C₁-C₆ peuvent prendre les positions désirées sur le cycle ;
R² représentent indépendamment les uns des autres, un atome d'hydrogène, d'halogène, des groupements alkyle en C₁-C₁₈, aryloxy, arylthio, hétaryloxy ou hétarylthio, pouvant chacun être substitué par des groupements alkyle en C₁-C₁₀, alcoxy en C₁-C₆, cyano ou carboxy.

8. Imides de pérylène-3,4-dicarboxylique de formule générale Ia selon la revendication 7, dans lesquels les variables prennent les significations suivantes :
R^{1'} représente un groupement alkyle en C₁₄-C₃₀, substitué une fois par un groupement carboxy, sulfo, hydroxy ou un reste hétérocyclique à 5-7 maillons relié par un atome d'azote et qui peut contenir d'autres hétéroatomes et être aromatique,
un groupement cycloalkyle en C₅-C₈ ;
un groupement phényle substitué une ou plusieurs fois par un groupement alkyle en C₁-C₄ dans les deux positions ortho au moins, un atome d'halogène, des groupements hydroxy, cyano,
carboxy, -COHNR⁴, -NHCOR⁴ et/ou une fois par un groupement aryl- ou hétarylazo, qui peuvent être substitués à chaque fois par des groupements alkyle en C₁-C₁₀, alcoxy en C₁-C₆, des atomes d'halogène, des groupements hydroxy, cyano ou carboxy,
un groupement hétaryle, pouvant être substitué par les substituants cités pour le groupement phényle et/ou par des groupements alcoxy en C₁-C₄, où les substituants alkyle en C₁-C₄ peuvent prendre les positions désirées sur le cycle ;
R² représentent indépendamment les uns des autres, un atome d'hydrogène, d'halogène, un groupement phénoxy, pouvant être substitué par des groupements alkyle en C₁-C₆, alcoxy en C₁-C₆, cyano ou carboxy.

9. Imides de pérylène-3,4-dicarboxylique de formule générale la selon la revendication 7, dans lesquels les variables prennent les significations suivantes :
R^{1'} représente un groupement alkyle en C₁₄-C₃₀, substitué une fois par des groupements carboxy, sulfo, ou hydroxy,
un groupement cycloalkyle en C₅-C₇ ;
un groupement phényle substitué une fois par un groupement alkyle en C₁-C₄ dans les deux positions ortho, des groupements hydroxy, cyano, carboxy, -CONHR⁴ ou -NHCOR⁴, où
R⁴ représente des groupements alkyle en C₁-C₄ ou phényle, pouvant être substitués par des groupements alkyle en C₁-C₄, alcoxy en C₁-C₄ ou cyano ;
ou par un groupement phényl- ou naphtylazo, pouvant être substitué par des groupements alkyle en C₁-C₄, alcoxy en C₁-C₄, hydroxy ou cyano ;
un groupement hétaryle monocyclique, pouvant être substitué une ou plusieurs fois par des groupements alkyle en C₁-C_{4,} alcoxy en C₁-C₄, hydroxy ou cyano ou une fois par un groupement phényl- ou naphtylazo, pouvant être substitué par des groupements alkyle en C₁-C₄, alcoxy en C₁-C₄, des groupements hydroxy ou cyano,
R² représentent indépendamment les uns des autres, un atome d'hydrogène ou un groupement phénoxy, pouvant être substitué par un groupement alkyle en C₁-C₄.

10. Utilisation d'imides de pérylène-3,4-dicarboxylique de formule générale la selon l'une quelconque des revendications 7 à 9 en tant que colorants fluorescents, pigments ou précurseurs d'additifs pour pigments.

## Revendications (Revendications pour l'(les) Etat(s) contractant(s) suivant(s): CH, DE, FR, GB, LI)

1. Procédé de préparation d'imides de pérylène-3,4-dicarboxylique par réaction d'un pérylène-3,4,9,10-tétracarboxylique ou de l'anhydride correspondant avec une amine primaire, caractérisé en ce que l'on effectue la réaction dans un milieu réactionnel ne contenant quasiment pas d'eau, en présence d'un composé basique azoté tertiaire en tant que solvant et d'un métal de transition ou d'un sel de métal de transition en tant que catalyseur.

2. Procédé selon la revendication 1, caractérisé en ce que l'on utilise un composé hétéroaromatique contenant de l'azote en tant que composé basique azoté tertiaire.

3. Procédé selon la revendication 1 ou 2, caractérisé en ce que l'on utilise en tant que catalyseur du zinc, des sels de zinc, du cuivre, des sels de cuivre ou des mélanges de ceux-ci.

4. Procédé de purification d'imides de pérylène-3,4-dicarboxylique obtenus par réaction d'un pérylène-3,4,9,10-tétracarboxylique ou de l'anhydride correspondant avec une amine primaire, caractérisé en ce que l'on chauffe tout d'abord les produits bruts dans de la N-méthylpyrrolidone puis en ce que l'on traite ensuite les adduits formés avec la N-méthylpyrrolidone avec une base, en présence d'un diluant organique, et l'on fait éventuellement subir aux produits isolés un traitement supplémentaire avec un acide aqueux.

5. Procédé selon la revendication 4, caractérisé en ce que l'on utilise en tant que base un hydroxyde de métal alcalin ou un alcoolate de métal alcalin.

6. Procédé de purification d'imides de pérylène-3,4-dicarboxylique obtenus par réaction d'un pérylène-3,4,9,10-tétracarboxylique ou de l'anhydride correspondant avec une amine primaire, caractérisé en ce que l'on effectue la réaction en présence d'un composé basique azoté tertiaire en tant que solvant et d'un métal de transition ou d'un sel de métal de transition en tant que catalyseur, et en ce que l'on chauffe tout d'abord les produits bruts dans de la N-méthylpyrrolidone puis en ce que l'on traite ensuite les adduits formés avec la N-méthylpyrrolidone avec une base, en présence d'un diluant organique, et l'on fait éventuellement subir aux produits isolés un traitement supplémentaire avec un acide aqueux.

7. Imides de pérylène-3,4-dicarboxylique de formule générale la 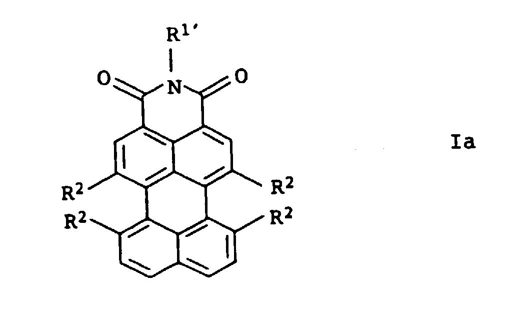 dans laquelle les variables ont la signification suivante :
R^{1'} représente un groupement alkyle en C₁₄-C₃₀, dont la chaîne carbonée peut être interrompue par un ou plusieurs groupements -O-, -S-, -NR³-, -CO- et/ou -SO₂- et pouvant être substitué une ou plusieurs fois par un groupement carboxy, sulfo, hydroxy, cyano, alcoxy en C₁-C₆, ou un reste hétérocyclique à 5-7 maillons relié par un atome d'azote et qui peut contenir d'autres hétéroatomes et être aromatique, où
R³ représente un atome d'hydrogène ou un groupement alkyle en C₁-C₆;
un groupement cycloalkyle en C₅-C₈ dont le squelette carboné peut être interrompu par un ou plusieurs groupements -O-, -S-, et/ou -NR³-;
un groupement phényle substitué une ou plusieurs fois par un groupement alkyle en C₁-C₄ ou méthoxy dans les deux positions ortho au moins, des groupements alkyle en C₅-C₁₈, alcoxy en C₂-C₆, des atomes d'halogène, des groupements hydroxy, cyano, carboxy, -CONHR⁴, -NHCOR⁴ et/ou aryl- ou hétarylazo, qui peuvent chacun être substitués par des groupements alkyle en C₁-C₁₀, alcoxy en C₁-C₆, des atomes d'halogène, des groupements hydroxy, cyano ou carboxy, où
R⁴ représente un atome d'hydrogène, un groupement alkyle en C₁-C₁₈, aryle ou hétaryle pouvant chacun être substitué par des groupements alkyle en C₁-C₆, alcoxy en C₁-C₆, des atomes d'halogène, des groupements hydroxy ou cyano ;
un groupement naphtyle ou hétaryle, pouvant chacun être substitué par les substituants cités pour le groupement phényle, où les substituants alkyle en C₁-C₄ et alcoxy en C₁-C₆ peuvent prendre les positions désirées sur le cycle ;
R² représentent indépendamment les uns des autres, un atome d'hydrogène, d'halogène, des groupements alkyle en C₁-C₁₈, aryloxy, arylthio, hétaryloxy ou hétarylthio, pouvant chacun être substitué par des groupements alkyle en C₁-C₁₀, alcoxy en C₁-C₆, cyano ou carboxy,
les imides de N-(1-tétradécyl)-, -(1-octylnonyl)-, -(1-nonyldécyl)- et -(cycloalkyle en C₅-C₈)pérylène-3,4-dicarboxylique étant exclus.

8. Imides de pérylène-3,4-dicarboxylique de formule générale la selon la revendication 7, dans lesquels les variables prennent les significations suivantes :
R^{1'} représente un groupement alkyle en C₁₄-C₃₀, substitué une fois par un groupement carboxy, sulfo, hydroxy ou un reste hétérocyclique à 5-7 maillons relié par un atome d'azote et qui peut contenir d'autres hétéroatomes et être aromatique,
un groupement cycloalkyle en C₅-C₈;
un groupement phényle substitué une ou plusieurs fois par un groupement alkyle en C₁-C₄ dans les deux positions ortho au moins, un atome d'halogène, des groupements hydroxy, cyano,
carboxy, -COHNR⁴, -NHCOR⁴ et/ou une fois par un groupement aryl- ou hétarylazo, qui peuvent être substitués à chaque fois par des groupements alkyle en C₁-C₁₀, alcoxy en
C₁-C₆, des atomes d'halogène, des groupements hydroxy, cyano ou carboxy,
un groupement hétaryle, pouvant être substitué par les substituants cités pour le groupement phényle et/ou par des groupements alcoxy en C₁-C₄, où les substituants alkyle en C₁-C₄ peuvent prendre les positions désirées sur le cycle ;
R² représentent indépendamment les uns des autres, un atome d'hydrogène, d'halogène, un groupement phénoxy, pouvant être substitué par des groupements alkyle en C₁-C₆, alcoxy en C₁-C₆, cyano ou carboxy,
où les imides de N-cyclopentyl-, -cyclohexyl-, -cycloheptyl- et -cyclooctylpérylène-3,4-dicarboxylique sont exclus.

9. Imides de pérylène-3,4-dicarboxylique de formule générale la selon la revendication 7, dans lesquels les variables prennent les significations suivantes :
R^{1'} représente un groupement alkyle en C₁₄-C₃₀, substitué une fois par des groupements carboxy, sulfo, ou hydroxy,
un groupement cycloalkyle en C₅-C₇ ;
un groupement phényle substitué une fois par un groupement alkyle en C₁-C₄ dans les deux positions ortho, des groupements hydroxy, cyano, carboxy, -CONHR⁴ ou -NHCOR⁴, où
R⁴ représente des groupements alkyle en C₁-C₄ ou phényle, pouvant être substitués par des groupements alkyle en C₁-C₄, alcoxy en C₁-C₄ ou cyano ;
ou par un groupement phényl- ou naphtylazo, pouvant être substitué par des groupements alkyle en C₁-C₄, alcoxy en C₁-C₄, hydroxy ou cyano ;
un groupement hétaryle monocyclique, pouvant être substitué une ou plusieurs fois par des groupements alkyle en C₁-C₄,
alcoxy en C₁-C₄, hydroxy ou cyano ou une fois par un groupement phényl- ou naphtylazo, pouvant être substitué par des groupements alkyle en C₁-C₄, alcoxy en C₁-C₄, des groupements hydroxy ou cyano,
R² représentent indépendamment les uns des autres, un atome d'hydrogène ou un groupement phénoxy, pouvant être substitué par un groupement alkyle en C₁-C₄,
où les imides de N-cyclopentyl-, -cyclohexyl- et -cydoheptylpérylène-3,4-dicarboxylique sont exclus.

10. Utilisation d'imides de pérylène-3,4-dicarboxylique de formule générale la selon l'une quelconque des revendications 7 à 9 en tant que colorants fluorescents, pigments ou précurseurs d'additifs pour pigments.
